(19) Europäisches Patentamt
European Patent Office
Office européen des brevets

(11) EP 0 906 912 B1

(12) **FASCICULE DE BREVET EUROPEEN**

(45) Date de publication et mention de la délivrance du brevet:
**08.01.2003 Bulletin 2003/02**

(21) Numéro de dépôt: **98402415.8**

(22) Date de dépôt: **01.10.1998**

(51) Int Cl.7: **C07D 319/18**, A61K 31/335, C07D 311/18, C07D 405/04, C07D 405/14, C07D 409/04, A61K 31/445, A61K 31/40

(54) **Nouveaux composés de l'indanol, leur procédé de préparation et les compositions pharmaceutiques qui les contiennent**

Indanol-Derivate, Verfahren zu deren Herstellung und diese enthaltende pharmazeutische Zusammensetzungen

Indanol derivatives, process for their preparation and pharmaceutical compositions containing them

(84) Etats contractants désignés:
**AT BE CH DE DK ES FI FR GB GR IE IT LI LU NL PT SE**

(30) Priorité: **03.10.1997 FR 9712336**

(43) Date de publication de la demande:
**07.04.1999 Bulletin 1999/14**

(73) Titulaire: **LES LABORATOIRES SERVIER**
**92200 Neuilly sur Seine (FR)**

(72) Inventeurs:
- **Peglion, Jean-Louis**
  **78110 le Vesinet (FR)**
- **Goument, Bertrand**
  **78220 Viroflay (FR)**
- **Millan, Mark**
  **78230 le Pecq (FR)**
- **Newman-Tancredi, Adrian**
  **78230 le Pecq (FR)**
- **Dekeyne, Anne**
  **78470 Saint Remy les Chevreuses (FR)**

(56) Documents cités:
**EP-A- 0 574 313**
**EP-A- 0 745 598**
**WO-A-87/02035**
**US-A- 5 194 437**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet de nouveaux composés de l'indanol, leur procédé de préparation et les compositions pharmaceutiques qui les contiennent.

Elle concerne plus spécialement :

- les composés de l'indanol de formule I:

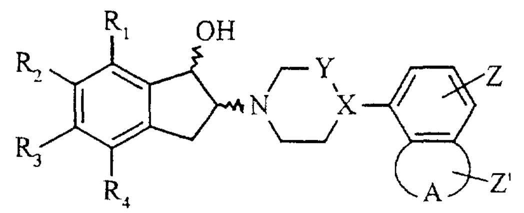

(I)

dans laquelle :

- ♦ $R_1$, $R_2$, $R_3$ et $R_4$ identiques ou différents, représentent chacun un atome d'hydrogène ou d'halogène, un radical ($C_1$-$C_5$)alkyle, ($C_2$-$C_5$)alkényle ou ($C_2$-$C_5$) alkynyle, chacun en chaîne linéaire ou ramifiée, un radical cycloalkylalkyle dans lequel la partie cycloalkyle renferme de 3 à 7 atomes de carbone et la partie alkyle de 1 à 5 atomes de carbone, un radical trifluorométhyle, CHO, COOH, COO($C_1$-$C_5$)alkyle, CO($C_1$-$C_5$)alkyle, $CH_2OH$, hydroxy, ($C_1$-$C_5$)alkoxy, ($C_2$-$C_5$) alkénoxy, ($C_2$-$C_5$)alkynoxy, benzyloxy, cyano, nitro,

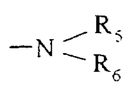 ou 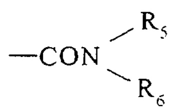

dans lesquels $R_5$ et $R_6$ identiques ou différents représentent chacun un atome d'hydrogène ou un radical ($C_1$-$C_5$) alkyle, -CO($C_1$-$C_5$)alkyle ou -COO($C_1$-$C_5$)alkyle; ou

- ♦ $R_1$, $R_2$, $R_3$ et $R_4$ pris deux à deux en position adjacente, forment avec les atomes de carbone du noyau phényle auxquels ils sont liés un cycle de 5 à 7 chaînons, renfermant une ou plusieurs double liaisons et composé d'atomes choisis parmi les atomes de carbone, oxygène, azote et soufre, et les groupements restants, n'étant pas pris deux à deux en position adjacente, représentent chacun un atome d'hydrogène,

- ♦ X-Y représente :
N-$CH_2$, C=CH, CH-$CH_2$ ou

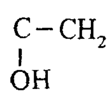 ;

- ♦ A forme avec les deux atomes de carbone du cycle phényle auxquels ils est lié un hétérocycle de 5 à 7 chaînons renfermant une ou plusieurs double liaisons et contenant un ou deux hétéroatomes, identiques ou différents, choisis parmi les atomes d'oxygène et soufre ;

- ♦ Z représente un atome d'hydrogène ou d'halogène, ou un radical hydroxy, ($C_1$-$C_5$)alkoxy, et

- ♦ Z' représente un atome d'hydrogène ou un radical oxo, hydroxy, ($C_1$-$C_5$)alkoxy ou $CH_2OH$,

sous forme d'isomères cis ou trans, chacun d'eux pouvant exister sous forme racémique ou optiquement active, et leurs sels d'addition avec un acide pharmaceutiquement acceptable.

Ainsi, dans la formule (I), le groupe

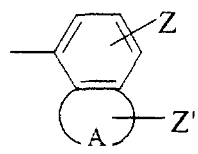

représente plus spécifiquement

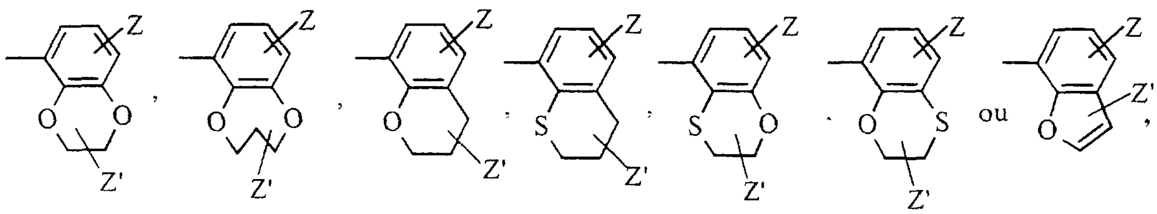

, , ; ; . ou ,

dans lesquels Z et Z' ont les significations précédemment définies.

Un grand nombre de sous-types réceptoriels de la sérotonine ont été identifiés à ce jour. A l'intérieur même de la classe des récepteurs 5-$HT_1$ les études de biologie moléculaire et de pharmacologie ont permis une subdivision entre cinq récepteurs différents, 5-$HT_{1A}$, 5-$HT_{1B}$, 5-$HT_{1D}$, 5-$HT_{1E}$, 5-$HT_{1F}$, (Humphrey, P.P.A. et al., A proposed new nomenclature for 5-HT receptors, *Trends in Pharmacological Sciences,* **1993**, 14, 233).

Des travaux précédents menés dans les services de la demanderesse (brevet U.S. 5.194.437) avaient permis de montrer que des composés du type :

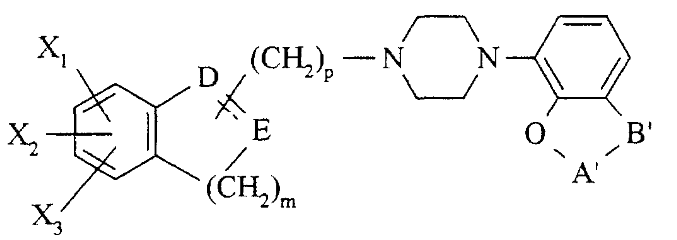

possédaient des propriétés antagonistes des récepteurs sérotoninergiques 5-$HT_{1A}$.

Les recherches conduites depuis lors dans les laboratoires de la demanderesse ont permis d'identifier des substances qui se différentient structuralement des précédentes par la présence d'une fonction alcool sur le cycle pentagonal du groupe indane, ce qui confère aux nouveaux composés une sécurité d'utilisation accrue, due à la meilleure sélectivité réceptorielle des produits de l'invention. En effet, il a ainsi été possible d'augmenter le ratio entre les affinités 5-$HT_{1A}$ et $\alpha_1$ d'une part et les affinités 5-$HT_{1A}$ et $D_2$ d'autre part, en minimisant les effets au niveau des sites $\alpha_1$ et $D_2$. Les nouveaux composés se comportent donc comme des ligands 5-$HT_{1A}$ beaucoup plus sélectifs et induisent de ce fait moins d'effets secondaires. L'état antérieur de la technique peut aussi être illustré par le brevet WO87/02035 qui revendique des amino-alcools cycliques de formule générale :

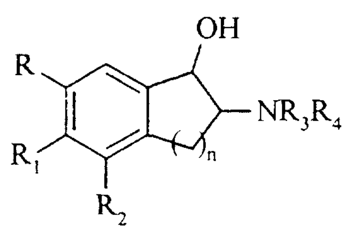

dans laquelle $NR_3R_4$ peut être :

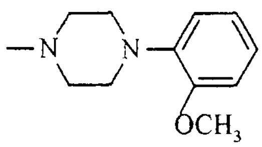

Toutefois ces dérivés sont utilisés dans le traitement des maladies cardiovasculaires (antihypertenseurs, antiaggrégants plaquettaires, spasmolytiques, etc...) et ne sauraient avoir inspirés les composés de la présente invention ni leur utilisation dans les domaines revendiqués ci-après.

Les études in vivo, réalisées avec les composés de la présente invention ont confirmé les résultats obtenus in vitro lors des études de liaisons et précisé le site d'action puisque les récepteurs $5HT_{1A}$ sont localisés aussi bien au niveau pré-synaptique que post-synaptique.

En effet, l'enregistrement de l'activité électrique unitaire, extracellulaire, dans le noyau dorsal du raphé chez le rat permet de préciser le caractère agoniste ou antagoniste $5\text{-}HT_{1A}$ au niveau pré-synaptique, tandis que le test de la température corporelle chez le rat permet de montrer le caractère agoniste ou antagoniste post-synaptique des produits de l'invention. Enfin le test des vocalisations ultrasoniques chez le rat renseigne efficacement sur les effets anxiolytiques des produits testés.

Ainsi les composés de la présente invention pourront être avantageusement utilisés dans les maladies du système nerveux central, notamment l'anxiété, la dépression, les psychoses, la schizophrénie, les troubles de la cognition, le stress, l'anorexie ainsi que dans le traitement des manifestations de la douleur.

La présente invention a également pour objet le procédé de préparation des composés de formule I caractérisé en ce que une cétone de formule II :

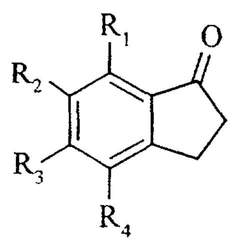

(II)

dans laquelle $R_1$, $R_2$, $R_3$ et $R_4$ ont les significations précédemment définies,
est halogénée en position $\alpha$ de la fonction cétone pour obtenir un composé de formule III :

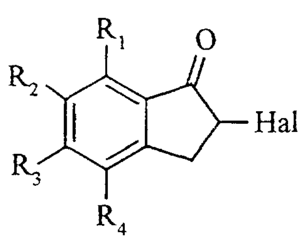

(III)

dans laquelle $R_1$, $R_2$, $R_3$, $R_4$ prennent les valeurs précédemment définies et Hal représente un atome de chlore, brome ou iode,
lequel composé III est mis à réagir avec une amine de formule IV :

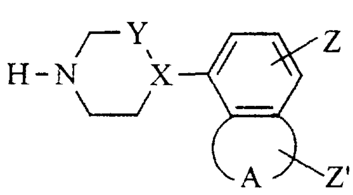

(IV)

dans laquelle X-Y, A, Z et Z' ont les significations précédemment définies, pour donner un composé de formule V :

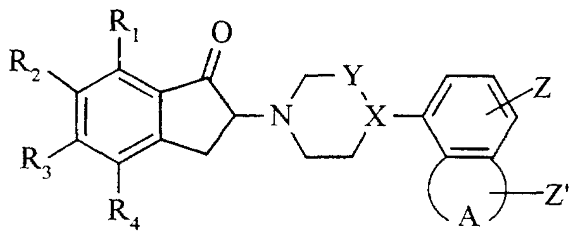

(V)

dans laquelle $R_1$, $R_2$, $R_3$, $R_4$, X-Y, A, Z et Z' ont les significations précédemment définies qui est réduit en un mélange d'amino-alcools de formules I trans et I cis:

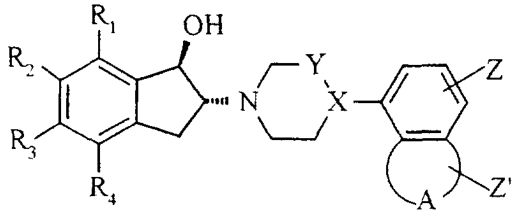

(I trans)

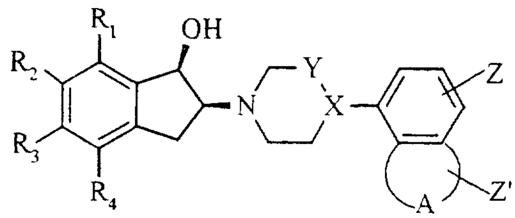

(I cis)

dans lesquelles $R_1$, $R_2$, $R_3$, $R_4$, X-Y, A, Z et Z' ont les significations précédemment définies.

Les composés I trans et I cis sont ensuite séparés par les méthodes classiques de séparation des composés organiques, c'est à dire par recristallisation fractionnée, séparation sur colonne de silice (flash chromatographie) ou séparation par C.L.H.P. (Chromatographie Liquide Haute Performance).

L'ensemble des composés I trans et I cis forme l'ensemble des composés de formule I.

Les composés de formule I dans laquelle X-Y représente C=CH ou $CH-CH_2$, c'est-à-dire les composés répondant plus spécifiquement à la formule Ia :

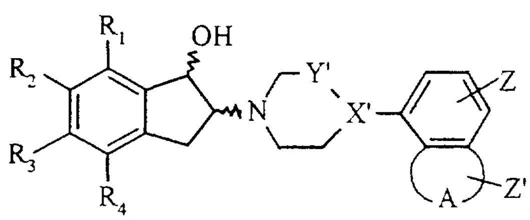

(Ia)

dans laquelle :

$R_1$, $R_2$, $R_3$, $R_4$, A, Z et Z' ont les significations précédemment définies et
X'-Y' représente C=CH ou $CH-CH_2$,

peuvent aussi être avantageusement préparés en utilisant la méthode suivante caractérisée en ce que l'on fait réagir un composé de formule VI :

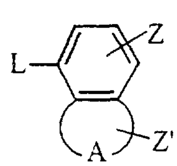

(VI)

dans laquelle :

A, Z et Z' ont les significations précédemment définies, et
L représente un groupe labile choisi parmi Br, I ou $OSO_2CF_3$,

- avec le diéthyl(pyrid-1-yl)borane dans les conditions de la réaction de Suzuki,
- pour obtenir un composé de formule VII :

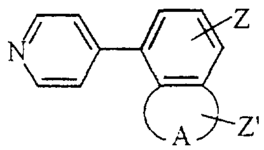

(VII)

dans laquelle A, Z et Z' ont les significations précédemment définies,

- que l'on fait réagir avec un composé de formule III précédemment définie, pour obtenir un composé de formule VIII :

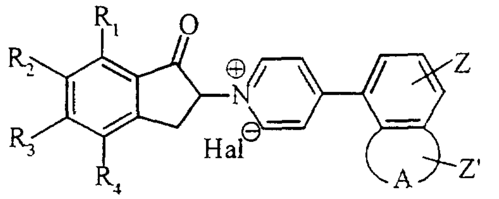

(VIII)

dans laquelle $R_1$, $R_2$, $R_3$, $R_4$, A, Z, Z' et Hal ont les significations précédemment définies,

- que l'on réduit par un borohydrure métallique dans un solvant alcoolique pour conduire à un mélange d'aminoalcools de formule I'a trans et I'a cis :

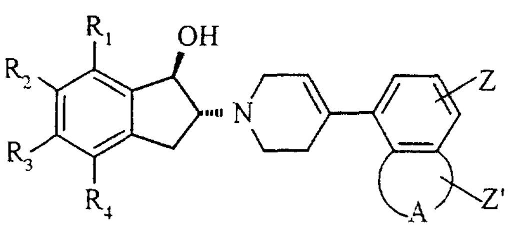

(I'a trans)

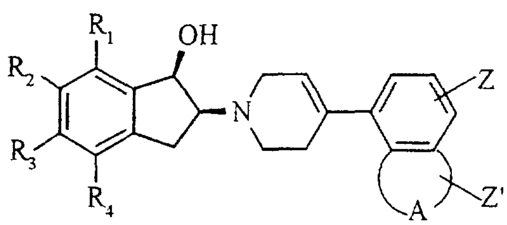

(I'a cis)

dans lesquelles $R_1$, $R_2$, $R_3$, $R_4$, A, Z et Z' ont les significations précédemment définies,

- lesquels aminoalcools sont, si on le désire, réduits à nouveau, par de l'hydrogène en présence d'un catalyseur approprié, en composés de formule I"a trans et I"a cis :

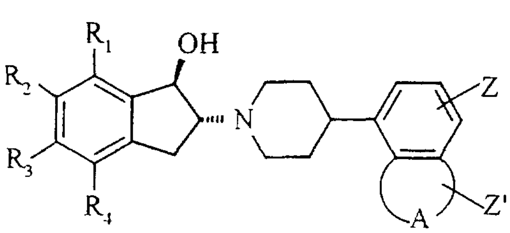

(I"a trans)

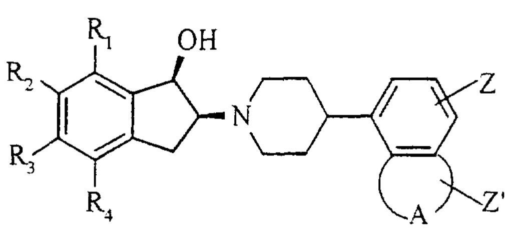

(I"a cis)

dans lesquelles $R_1$, $R_2$, $R_3$, $R_4$, A, Z et Z' ont les significations précédemment définies,

- et dans le cas où Z' prend la valeur oxo, il est judicieux de partir d'un composé VII dans lequel cette fonction oxo est protégée, par exemple sous forme de 1,3-dioxolanne, puis déprotégée soit au niveau des composés I'a trans et I'a cis soit au niveau des composés I"a trans et I"a cis,
- les composés I'a trans et I'a cis d'une part et I"a trans et I"a cis d'autre part, sont ensuite séparés par les méthodes classiques de séparation des composés organiques, c'est-à-dire par recristallisation fractionnée, séparation sur colonne de silice ou séparation par C.L.H.P.

L'ensemble des composés I'a trans, I'a cis, I"a trans et I"a cis forme l'ensemble des composés de formule Ia, laquelle est incluse dans la formule I.

L'halogénation du composé II est une réaction classique qui peut être réalisée grâce à un grand nombre de réactifs. Dans le cas où l'atome d'halogène est un atome de brome, cette réaction peut être avantageusement réalisée au moyen de tribromure de tétrabutyl ammonium dans le méthanol et le chlorure de méthylène.

La réaction du composé III avec le composé IV s'effectue, entre autre, avantageusement dans le diméthylformamide en présence de $K_2CO_3$.

Les matières premières de formule II et de formule VI, lorsqu'elles ne sont pas commerciales, ont été préparées à partir de substances connues, selon des procédés décrits dans la littérature.

Les formes optiquement actives des composés de formule I ont été obtenues par dédoublement des formes racémiques des composés de formule I ou de leurs analogues estérifiés au niveau de la fonction alcool de l'indanol, selon des méthodes connues de la littérature.

La présente invention a également pour objet les compositions pharmaceutiques contenant comme principe actif au moins un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par voie orale, rectale ou parentérale.

La posologie peut varier selon l'âge et le poids du patient, la voie d'administration, la nature de la maladie et les traitements associés et s'échelonne de 0,5 à 25 mg de principe actif, 1 à 3 fois par jour.

Les exemples suivants, donnés à titre non-limitatif, illustrent la présente invention. Les points de fusion (PF) ont été déterminés soit à la platine chauffante de Kofler (K), soit à la platine chauffante sous microscope (MK).

**EXEMPLE 1**

<u>Cis-2-[4-(2,3-dihydro[1,4]benzodioxin-5-yl)pipérazin-1-yl]-6-méthoxyindan-1-ol</u>

**Stade 1 :** 2-bromo-6-méthoxy indan-1-one

A 16,2 g (100 mmol.) de 6-méthoxy indan-1-one en solution dans 400 ml de méthanol et 1 litre de dichlorométhane, à température ambiante, on ajoute par fraction en un quart d'heure 49,2 g (102 mmol.) de tribromure de tétra-n-butyl ammonium, puis agite pendant la nuit à température ambiante. Après évaporation, on reprend par 500 ml de dichlorométhane et lave deux fois par 250 ml d'acide chlorhydrique N. Après séchage sur sulfate de magnésium et concentration, puis filtration rapide sur 500 g de silice (éluant : dichlorométhane), on obtient 23,4 g du produit désiré. Rendement : 97 %

**Stade 2 :** 2-[4-(2,3-dihydro[1,4]benzodioxin-5-yl)pipérazin-1-yl]-6-méthoxy indan-1-one

A température ambiante, on agite pendant 20 heures une suspension préparée à partir de 25,3 g (100 mmol.) du composé obtenu au stade 1, 22,0 g (100 mmol.) de (2,3-dihydro [1,4]benzodioxin-5-yl) pipérazine, 13,8 g (100 mmol.) de carbonate de potassium et 140 ml de diméthylformamide. On verse ensuite dans 1,5 litre d'eau, filtre le solide formé (violet), le rince à l'eau et le sèche sous vide pour obtenir 28,1 g du produit attendu. Rendement : 74 %

**Stade 3 :** produit titre

A 28,0 g (73,6 mmol.) du composé obtenu au stade 2 dans 220 ml de tétrahydrofurane, à température ambiante, on ajoute par fractions en 15 mn, 2,8 (73,8 mmol.) de borohydrure de sodium. On agite la nuit à température ambiante, puis 4 heures à reflux. Après évaporation, le résidu est repris par 1 litre de dichlorométhane et lavé deux fois par 500 ml d'eau puis séché sur sulfate de magnésium. Après concentration, le résidu est chromatographié sur silice (éluant : dichlorométhane/méthanol 99/1 puis 98/2). Le produit élué en premier correspond à l'isomère cis. Sa

stéréochimie a été démontrée par IR, en solution dans le chloroforme, selon la méthode décrite par H.-J. Rimek et al. (*Justus Liebigs Ann. Chem.,* **1969,** 726, 25-29).
IR($CHCl_3$) : $\nu_{OH\ lié}$ : 3380 $cm^{-1}$ (bande large)
PF(MK) : 207-210°C.

**EXEMPLE 2**

Trans-2-[4-(2,3-dihydro[1,4]benzodioxin-5-yl)pipérazin-1-yl]-6-méthoxyindan-1-ol

Le produit élué en second lors de la chromatographie effectuée au stade 3 de l'exemple précédent correspond au produit titre. Sa stéréochimie a été démontrée selon la même méthode que précédemment.
IR($CHCl_3$) : $\nu_{OH\ libre}$ : 3580 $cm^{-1}$ (bande fine)
PF(MK) : 145-148°C.

**EXEMPLE 3**

Cis-2-[4-(2,3-dihydro[1,4]benzodioxin-5-yl)pipérazin-1-yl]-5-méthoxyindan-1-ol

Préparé de la même façon que le produit de l'exemple 1, mais en utilisant au stade 1 la 5-méthoxy indan-1-one à la place de la 6-méthoxy indan-1-one.
IR($CHCl_3$) : $\nu_{OH\ lié}$ : 3400 $cm^{-1}$ (bande large)
PF(MK) : 214-217°C.

**EXEMPLE 4**

Trans-2-[4-(2,3-dihydro[1,4]benzodioxin-5-yl)pipérazin-1-yl]-5-méthoxyindan-1-ol

Préparé de la même façon que le produit de l'exemple 2, mais en utilisant au stade 1 de l'exemple 1 la 5-méthoxy indan-1-one à la place de la 6-méthoxy indan-1-one.
IR($CHCl_3$) : $\nu_{OH\ libre}$ : 3700 $cm^{-1}$ et 3600 $cm^{-1}$ (bandes fines)
PF(MK) : 137-140°C.

**EXEMPLE 5**

Cis-2-[4-(2,3-dihydro[1,4]benzodioxin-5-yl)pipérazin-1-yl]indan-1-ol

Préparé de la même façon que le produit de l'exemple 1, mais en utilisant au stade 1 l'indan-1-one à la place de la 6-méthoxy indan-1-one.
IR($CHCl_3$): $\nu_{OH\ lié}$ : 3400 $cm^{-1}$ (bande large)
PF(MK): 196-198°C.

**EXEMPLE 6**

Trans-2-[4-(2,3-dihydro[1,4]benzodioxin-5-yl)pipérazin-1-yl]indan-1-ol

Préparé de la même façon que le produit de l'exemple 2, mais en utilisant au stade 1 de l'exemple 1 l'indan-1-one à la place de la 6-méthoxy indan-1-one.
IR($CHCl_3$) : $\nu_{OH\ libre}$ : 3600 $cm^{-1}$ (bande fine)
PF(MK):211-214°C

**EXEMPLE 7**

(+)-Trans-2-[4-(2,3-dihydro[1,4]benzodioxin-5-yl)pipérazin-1-yl]indan-1-ol

**Stade 1** : (±) -Trans-1-(1-acétoxyindan -2-yl)-4-(2,3-dihydro[1,4]benzodioxin-5-yl) pipérazine

4,9 g du produit obtenu à l'exemple 6 sont traités, à température ambiante pendant une nuit, par 706 ml d'anhydride acétique et 141 ml de pyridine. Après concentration du milieu réactionnel, on reprend par de l'eau et du

chlorure de méthylène. Après décantation, la phase organique est lavée, séchée, évaporée et purifiée par chromatographie sur colonne de silice pour conduire à 5,2 g de produit attendu.

**Stade 2** : Séparation des isomères optiques

Les 5,2 g de produit obtenus au stade précédent sont séparés par C.L.H.P., sur une phase chirale. Un éluant préparé à partir d'un mélange isooctane/éthanol/diéthylamine permet d'isoler 1,8 g d'un composé qui correspond au premier pic, pur, le deuxième isomère (1,1 g) correspondant au deuxième pic, pur, est obtenu grâce à un éluant composé d'un mélange n-heptane/éthanol/diéthylamine.

**Stade 3** : produit titre

Les 1,8 g du premier produit obtenu lors de la séparation chromatographique du stade 2 sont traités par 1,28 g d'hydroxyde de potassium, 18 ml d'eau et 160 ml de méthanol, à reflux pendant 2 heures. On concentre, reprend au chlorure de méthylène, lave à l'eau, sèche et évapore pour conduire après recristallisation du résidu à 0,44 g d'un produit qui fond à 184-186°C (M.K.) et qui correspond à la structure attendue avec un excès énantiomérique de 99 %.
$[\alpha]^{20°C}$ (C = 0,5 % dans $CH_3OH$) :

| λ nm | 589 | 578 | 546 | 436 | 365 |
|---|---|---|---|---|---|
| α° | + 22,1 | + 22,9 | + 26,1 | + 47,2 | + 79,6 |

**EXEMPLE 8**

(-)-Trans-2-[4-(2,3-dihydro[1,4]benzodioxin-5-yl)pipérazin-1-yl]indan-1-ol

Les 1,1 g du deuxième produit obtenu lors de la séparation chromatographique sont traités de la même façon qu'au cours du stade 3 de l'exemple 7 pour conduire à 0,27 g d'un produit qui fond à 186-189°C (M.K.) et qui correspond à la structure attendue avec un excès énantiomérique supérieur à 97 %.
$[\alpha]^{20°C}$ (C = 0,5 % dans $CH_3OH$) :

| λ nm | 589 | 578 | 546 | 436 | 365 |
|---|---|---|---|---|---|
| α° | - 20,9 | - 22,1 | - 25,4 | - 45,7 | - 77,1 |

**EXEMPLE 9**

Cis-2-[4-(2,3-dihydro[1,4]benzodioxin-5-yl)pipérazin-1-yl]-6-hydroxyindan-1-ol

0,77 g du produit de l'exemple 1 est mis en suspension à -20°C dans le chlorure de méthylène. On y ajoute goutte à goutte 4,1 ml d'une solution (1M dans le chlorure de méthylène) de tribromure de bore. On agite 3 heures à -20°C puis laisse le mélange revenir à température ambiante et laisse 3 jours sous agitation. On filtre le précipité obtenu et le lave au chlorure de méthylène. On le concrétise dans l'eau pour obtenir 0,58 g d'un produit qui correspond au bromhydrate du produit attendu. Après libération du sel par une solution de bicarbonate de sodium, extraction par du chlorure de méthylène, évaporation et concrétisation, on obtient 0,25 g du produit attendu.
En opérant comme dans l'exemple 1, ont été préparés les produits, objet des exemples suivants :

**EXEMPLE 10**

Cis-2-[4-(2,3-dihydro[1,4]benzodioxin-5-yl)pipérazin-1-yl]-5-fluoroindan-1-ol

PF (MK) = 210-213°C

**EXEMPLE 11**

Cis-2-[4- (2,3-dihydro[1,4]benzodioxin-5-yl)pipérid-1-yl ]-5-fluoroindan-1-ol

PF (MK) = 197-201°C

**EXEMPLE 12**

Cis-2-[4-(3,4-dihydro-*2H*-chromén-8-yl)pipérazin-1-yl]-5-fluoroindan-1-ol

PF (MK) = 209-210°C

**EXEMPLES 13 A 16**

De la même façon que le produit de l'exemple 2, ont été préparés les produits, objet des exemple suivants:

**EXEMPLE 13**

Trans-2-[4-(2,3-dihydro[1,4]benzodioxin-5 -yl)pipérazin-1-yl]-5-fluoroindan-1-ol

PF (MK) = 220-224°C

**EXEMPLE 14**

Trans-2-[4-(2,3-dihydro[1,4]benzodioxin-5-yl)pipérid-1-yl]-5-fluoroindan-1-ol

PF (MK) = 204-209°C

**EXEMPLE 15**

Trans-2-[4-(3,4-dihydro-2H-chromén-8-yl)pipérazin-1-yl]-5-fluoroindan-1-ol

PF (MK) = 148-150°C

**EXEMPLE 16**

Trans-2-{4-[2,3-dihydro-2-(hydroxyméthyl)[1,4]-benzodioxin-5-yl]pipérazin-1-yl}-6-méthoxyindan-1-ol

**EXEMPLE 17**

Trans-2-{4-[7-Chloro-2,3-dihydro-2-(hydroxyméthyl)[1,4]-benzodioxin-5-yl]pipérazin-1-yl}-5-fluoroindan-1-ol

**EXEMPLE 18**

Cis-2-[4-(2,3-dihydro[1,4]benzodioxin-5-yl)-1,2,3,6-tétrahydropyrid-1-yl]indan-1-ol

**Stade 1 :** 4-(2,3-dihydro[1,4]benzodioxin-5-yl)pyridine

A température ambiante, on mélange 14,7 mmole de diéthyl(pyrid-4-yl)borane, 22 mmole de 5-bromo-2,3-dihydro[1,4]benzodioxine, 44,1 mole de KOH en poudre, 7,4 mmole de bromure de tétrabutylammonium et 0,74 mmoles de pallaidum tetrakis (triphénylphosphine) dans 75 ml de tétrahydrofurane, puis on porte à reflux pendant 24 heures. On ajoute 225 ml d'acétate d'éthyle et lave par une solution saturée en chlorure de sodium. On sèche sur $MgSO_4$, évapore et purifie sur colonne de silice (éluant $CH_2Cl_2$ puis $CH_2Cl_2/CH_3COOC_2H_5$, 80/20).
Rendement : 52 %

**Stade 2 :** Bromure de 4-(2,3-dihydro[1,4]benzodioxin-5-yl)-1-(indan-1-one-2-yl) pyridinium

2,41 g du produit obtenu au stade précédent et 2,45 g de 2-bromo-indan-1-one sont dissout dans 35 ml d'acétone et portés au reflux pendant 24 heures. On refroidit, filtre le solide, le rince à l'acétone et le sèche sous vide. On obtient ainsi 2,7 g de produit qui correspond à la structure attendue.

**Stade 3** : produit titre

A température ambiante, sur 2,6 g du produit obtenu au stade précédent en suspension dans 50 ml de méthanol, on ajoute par fractions, en 30 minutes, 1,18 g de borohydrure de sodium (très forte exothermie) puis on agite 30 minutes à température ambiante. On ajoute alors 2,8 ml d'acide acétique et évapore à sec. On reprend par 50 ml de soude N et extrait deux fois par 50 ml de chlorure de méthylène. Les phases organiques sont lavées à l'eau, séchées, évaporées et chromatographiées sur silice, éluant $CH_2Cl_2/CH_3COOC_2H_5$, 90/10 puis $CH_2Cl_2/CH_3OH$, 99/1. Le premier produit élué est recristallisé de l'éthanol pour conduire à 0,27 g de produit attendu.
PF(MK)= 109-112°C
La stéréochimie cis du produit est démontrée par infrarouge, en solution dans chloroforme, selon la méthode décrite par H.J. Rimek et al. (Justus Liebigs Ann. Chem., **1969,** 726, 25-20).
IR($CHCl_3$) : $\nu_{OH\ lié}$ : 3400 $cm^{-1}$ (bande large)

**EXEMPLE 19**

Trans-2-[4-(2,3-dihydro[1,4]benzodioxin-5-yl)-1,2,3,6-tétrahydropyrid-1-yl]indan-1-ol

Le produit élué en second lors de la chromatographie effectuée au stade 3 de l'exemple précédent correspond au produit titre.
PF (MK) = 182-184°C
Sa stéréochimie a été démontrée selon la méthode utilisée à l'exemple précédent. IR($CHCl_3$) : $\nu_{OH\ libre}$ : 3600 $cm^{-1}$ (bande fine)

**EXEMPLE 20**

Trans-2-[4-(2,3-dihydro[1,4]benzodioxin-5-yl)-1,2,3,6-tétrahydropyrid-1-y]-5-fluoroindan-1-ol

Préparé en utilisant successivement les méthodes décrites aux exemples 18 et 19 mais en utilisant au stade 2 de l'exemple 18 la 2-bromo-5-fluoroindan-1-one à la place de la 2-bromoindan-1-one.
Le produit titre a un point de fusion (MK) = 198-202°C

**EXEMPLE 21**

Trans-2-[4-(3,4-dihydro-*2H*-thiochromén-8-yl)-1,2,3,6-tétrahydropyrid-1-yl]-5-fluoroindan-1-ol

Préparé en utilisant successivement les méthodes décrites aux exemples 18 et 19 mais en utilisant au stade 1 de l'exemple 18 le 3,4-dihydro-*2H*-thiochromén-8-yl triflate à la place de la 5-bromo 2,3-dihydro[1,4]benzodioxine et au stade 2 de l'exemple 18 la 2-bromo-5-fluoro-indan-1-one à la place de la 2-bromo-indan-1-one.
Le produit titre a un point de fusion (MK) = 143-147°C

**EXEMPLE 22**

Trans-2-[4-(3,4-dihydro-*2H*-chromén-8-yl)-1,2,3,6-tetrahydropyrid-1-yl]-5-fluoroindan-1-ol

Préparé en utilisant successivement les méthodes décrites aux exemples 18 et 19 mais en utilisant au stade 1 de l'exemple 18 le 8-bromo-3,4-dihydro-*2H*-chromène et au stade 2 de l'exemple 18 la 2-bromo-5-fluoro-indan-1-one.

**EXEMPLE 23**

Trans-2-[4-(2,3-dihydro[1,4]benzodioxin-5-yl)pipérid-1-yl]indan-1-ol

0,44 g du produit obtenu à l'exemple 19 dissout dans 20 ml de $CH_3OH$ est hydrogéné à température ambiante et pression atmosphérique, en présence d'oxyde de platine. Après purification sur colonne de silice et recristallisation de l'acétonitrile, on obtient 0,17 g d'un produit dont la structure correspond à celle du produit attendu.
PF(MK)= 173-174°C

**EXEMPLE 24**

Trans-2-[4-(benzofuran-7-yl)pipérid-1-yl]-5 -fluoroindan-1-ol

Préparé en utilisant successivement les méthodes décrites aux exemples 18, 19 et 23, mais en utilisant au

stade 1 de l'exemple 18 le 7-bromobenzofurane à la place de la 5-bromo-2,3-dihydro[1,4]benzodioxine, et au stade 2 de l'exemple 18 la 2-bromo-5-fluoroindan-1-one à la place de la 2-bromo-indan-1-one.
Le produit titre a un point de fusion (MK) = 170-172°C

**EXEMPLE 25**

Trans-2-[4-(2,3-dihydro[1,4]benzodioxin-5-yl)pipérid-1-yl]-6-fluoroindan-1-ol

Préparé en utilisant successivement les méthodes décrites aux exemples 18, 19 et 23 mais en utilisant au stade 2 de l'exemple 18 la 2-bromo-6-fluoro-indan-1-one à la place de la 2-bromo-indan-1-one.
Le produit titre a un point de fusion (MK) = 159-163°C

**EXEMPLE 26**

Trans-2-[4-(2,3-dihydro[1,4]benzodioxin-5-yl)pipérid-1-yl]-5-méthylindan-1-ol

Préparé en utilisant successivement les méthodes décrites aux exemples 18, 19 et 23, mais en utilisant au stade 2 de l'exemple 18 la 2-bromo-5-méthyl-indan-1-one
Le produit titre a un point de fusion (MK) = 191-192°C

**EXEMPLE 27**

Trans-2-[4-(2,3-dihydro[1,4]benzodioxin-5-yl)pipérid-1yl]-6-méthylindan-1-ol

Préparé en utilisant successivement les méthodes décrites aux exemples 18, 19 et 23, mais en utilisant au stade 2 de l'exemple 18 la 2-bromo-6-méthylindan-1-one.
Le produit titre a un point de fusion (MK) = 225-226°C

**EXEMPLE 28**

Trans-2-[4-(2,3-dihydro[1,4]benzodioxin-5-yl)pipérid-1-yl]-5,6 -difluoroindan-1-ol

Préparé en utilisant successivement les méthodes décrites aux exemples 18, 19 et 23 mais en utilisant au stade 2 de l'exemple 18 la 2-bromo-5,6-difluoroindan-1-one.
Le produit titre a un point de fusion (MK) = 167-169°C

**EXEMPLE 29**

Trans-2-[4-(3,4-dihydro-6-fluoro-*2H*-chromén-8-yl)pipérid-1-yl]-5-fluoroindan-1-ol

Préparé en utilisant successivement les méthodes décrites aux exemples 18, 19 et 23 mais en utilisant au stade 1 de l'exemple 18 le 3,4-dihydro-*2H*-6-fluorochromén-8-yl triflate à la place de la 5-bromo-2,3-dihydro[1,4]benzodioxine et au stade 2 de l'exemple 18 la 2-bromo-5-fluoroindan-1-one à la place de la 2-bromoindan-1-one.
Le produit titre a un point de fusion (MK) = 160-162°C

**EXEMPLE 30**

Trans-2-[4-(2,3-dihydro[1,4]benzodioxin-5-yl)pipérid-1-yl]-5,6-méthylènedioxyindan-1-ol

Préparé en utilisant successivement les méthodes décrites aux exemples 18, 19 et 23 mais en utilisant au stade 2 de l'exemple 18 la 2-bromo-5,6-méthylènedioxyindan-1-one.
Le produit titre a un point de fusion (MK) = 212-215°C

**EXEMPLE 31**

Trans-2-[4-(2,3-dihydrobenzofuran-7-yl)pipérid-1-yl]-5-fluoroindan-1-ol

Préparé en utilisant successivement les méthodes décrites aux exemples 18, 19 et 23 mais en utilisant au

stade 1 de l'exemple 18 le 7-bromo-2,3-dihydrobenzofurane et au stade 2 de l'exemple 18 la 2-bromo-5-fluoroindan-1-one.
Le produit titre a un point de fusion (MK) = 215-217°C

**EXEMPLE 32**

Trans-2-[4-(2,3-dihydro[1,4]benzodioxin-5-yl)-4-hydroxypipérid-1-yl]-5-fluoroindan-1-ol

Préparé en utilisant successivement les méthodes décrites dans les exemples 1 et 2 mais en utilisant au stade 1 de l'exemple 1 la 5-fluoroindan-1-one et au stade 2 de l'exemple 1 la 4-(2,3-dihydro[1,4]benzodioxin-5-yl)-4-hydroxy pipéridine.

**EXEMPLES 33 A 42**

En opérant comme décrit dans les exemples 24 à 31, ont été préparés les produits, objet des exemples suivants :

**EXEMPLE 33**

Trans-2-[4-(7-chloro-2,3-dihydro[1,4]benzodioxin-5-yl)pipérid-1-yl]-5-fluoroindan-1-ol

PF(MK)= 175-177°C

**EXEMPLE 34**

Trans-2-[4-(3,4-dihydro-*2H*-chromén-8-yl)pipérid-1-yl]-5-fluoroindan-1-ol

**EXEMPLE 35**

Trans-6-bromo-2-[4-(3,4-dihydrobenzodioxin-5-yl)pipérid-1-yl]indan-1-ol

**EXEMPLE 36**

Trans-6-cyano-2-[4-(2,3-dihydro[1,4]benzodioxin-5-yl)pipérid-1-yl]indan-1-ol

**EXEMPLE 37**

Trans-6-carbamoyl-2-[4-(2,3-dihydro[1,4]benzodioxin-5-yl)pipérid-1-yl]indan-1-ol

**EXEMPLE 38**

Trans-2-[4-(2,3-dihydro[1,4]benzodioxin-5-yl)pipérid-1-yl]-6-trifluorométhylindan-1-ol

**EXEMPLE 39**

Trans-2-[4-(3,4-dihydro[1,4]-*2H*-chromén-8-yl)pipérid-1-yl]indan-1-ol

**EXEMPLE 40**

Trans-2-{4-[2,3-dihydro-2-(hydroxyméthyl)[1,4]benzodioxin-5-yl]pipérid-1-yl}-5-fluoroindan-1-ol

**EXEMPLE 41**

Trans-2-{4-[(3,4-dihydro)-4-oxo-*2H*-chromén-8-yl]pipérid-1-yl}-5-fluoroindan-1-ol,

En utilisant de plus, dans ce cas, la méthode de protection-déprotection de la fonction oxo.

**EXEMPLE 42**

Trans-2-{4-[(3,4-dihydro)-4-hydroxy-*2H*-chromén-8-yl]pipérid-1-yl}-5-fluoroindan-1-ol

**EXEMPLE 43 : ETUDE PHARMACOLOGIQUE**

A. Etude in vitro

• Détermination de l'affinité pour les récepteurs humains h5-$HT_{1A}$

L'affinité a été déterminée par des expériences de compétition avec le [$^3H$]-8-OH-DPAT (NEN, Les Ulis, Fance). Les membranes préparées à partir de cellules CHO transfectées avec le récepteur humain 5-$HT_{1A}$ ont été préparées comme décrit (Newman-Tancredi et al., *Neuropharmacol.*, **1997,** 36, 451-459). Les membranes sont incubées en triple avec 0,4 nM de [$^3H$]-8-OH-DPAT et le ligand froid dans un volume final de 1.0 ml, pendant deux heures et demie à 25°C. Le tampon d'incubation contient 25 mM de HEPES-NaOH (pH 7,4) et 5 mM de $MgCl_2$. La fixation non spécifique est déterminée avec 10 µM de 5-HT. A la fin de l'incubation, le milieu d'incubation est filtré au travers de filtres WHATMAN GF/B imprégnés avec 0,1 % de polyéthylénimine et lavés trois fois avec 5 ml de tampon refroidi. La radioactivité retenue sur les filtres est déterminée par comptage du liquide de scintillation. Les isothermes de binding sont analysées par régression non linéaire utilisant le logiciel 'PRISM' (GraphPad Software Inc., S. Diego, USA) pour déterminer les valeurs d'$IC_{50}$. Elles sont converties en constante de dissociation ($K_i$) par l'intermédiaire de l'équation de Cheng-Prusoff :

$$K_i = IC_{50} / (1 + L/K_d)$$

dans laquelle L est la concentration de [$^3H$]-8-OH-DPAT et $K_d$ est la constante de dissociation de [$^3H$]-8-OH-DPAT du récepteur humain h5-$HT_{1A}$ (0,65 nM).

A titre d'exemple et pour illustrer l'activité des produits de l'invention, le produit de l'exemple 6 à un $K_i$ de 1.73 nM.

• Détermination de l'affinité pour les récepteurs $\alpha_1$ de rat

L'affinité a été déterminée par des expériences de compétition avec le [$^3H$]-prazosin (NEN, Les Ulis, France). Les membranes sont préparées à partir de cortex frontal de rat comme décrit (Millan et al., *J. Pharmacol. Exp. Ther*, **1995**, 275, 885-898). Les membranes sont incubées en triple avec 0.2 nM de [$^3H$]-8-OH-DPAT et le ligand froid dans un volume final de 1.0 ml, pendant 60 minutes à 25°C. Le tampon d'incubation contient 50 nM de TRIS-HCl (pH 7,4), 4 mM de $CaCl_2$, 0,1 % (p/v) d'acide ascorbique et 10 µM de pargyline. La fixation non spécifique est déterminée avec 10 µM de phentolamine. A la fin de l'incubation, le milieu d'incubation est filtré au travers de filtres WHATMAN GF/B imprégnés avec 0,1 % de polyéthylénimine et lavés trois fois avec 5 ml de tampon refroidi. La radioactivité retenue sur les filtres est déterminée par comptage du liquide de scintillation. Les isothermes de binding sont analysées par régression non linéaire utilisant le logiciel 'PRISM' (GraphPad Software Inc., S. Diego, USA) pour déterminer des valeurs de $IC_{50}$. Elles sont converties en constante de dissociation ($K_i$) par l'intermédiaire de l'équation de Cheng-Prusoff :

$$K_i = IC_{50} / (1 + L/K_d)$$

dans laquelle L est la concentration de [$^3H$]-prazosin et $K_d$ est la constante de dissociation de [$^3H$]-prazosin du récepteur $\alpha_1$ (0,1 nM).

A titre d'exemple et pour illustrer l'activité des produits de l'invention, le produit de l'exemple 6 à un $K_i$ de 932 nM.

Ces deux résultats illustrent bien l'excellente sélectivité des produits de l'invention pour le récepteur 5-$HT_{1A}$ vis à vis du récepteur $\alpha_1$, ce qui doit conduire à des produits dépourvus d'effets secondaires cardiovasculaires et présentant ainsi une meilleure sécurité d'emploi.

B. Etude in vivo

1. Enregistrement "in vivo" de l'activité éléctrique unitaire, extracellulaire, dans le noyau dorsal du raphé chez le rat (récepteurs 5-$HT_{1A}$ pré-synaptiques)

• Principe

L'administration d'un agoniste sérotoninergique 5-$HT_{1A}$ diminue d'une manière dose-dépendante la fréquence de décharge des neurones. Cet effet est réversé par l'antagoniste sélectif 5-$HT_{1A}$, WAY 100,635.

• Méthode

Les rats sont anesthésiés à l'hydrate de chloral (400 mg/kg, i.p.) et placés dans un appareil de stéréotaxie (Unimécanique, France) après cathétérisation de la veine fémorale. Le niveau d'anesthésie est maintenu par une administration i.p. d'hydrate de chloral toutes les heures ; la température rectale est conservée à 37 ± 1°C par une couverture chauffante thermostatée. Une microélectrode en tungstène (10 MΩ, 1 μM) est descendue à l'aide d'un microdescendeur électronique (Unimécanique, France) au niveau du noyau dorsal du raphé (AP : -7,8 / bregma ; L : 0,0 ; H : 5,0-6,5/dura; atlas de Paxinos et Watson, 1986). Les potentiels de cellules sérotoninergiques sont caractérisées par leur morphologie (potentiels biphasiques positif-négatif, d'une durée inférieure à 2,5 msec) et leur rythme de décharge lent et régulier compris entre 0,5 et 2,5 Hz. On enregistre une seule cellule par animal.
Après une période ≥ 5 mn (activité basale) et une première injection de véhicule (eau distillée additionnée de quelques gouttes d'acide lactique dilué, pH ramené à 5 par NaOH 1N), le produit de l'invention est administré par voie intraveineuse en doses cumulées croissantes avec un intervalle de 5 minutes.

• Analyse des résultats

L'acquisition des données est faite par le logiciel Spike2 (Cambridge Electronic Design, England). La fréquence de décharge est mesurée sur une minute au maximum de variation entre chaque injection et exprimée en pourcentage de variation par rapport à l'activité basale (moyenne des cinq minutes précédant le premier traitement) définie comme 100 %. Le calcul de l'$ID_{50}$ est effectuée par une méthode de régression linéaire simple avec mesures répétées.

• Résultats

A titre d'exemple le tableau suivant montre les effets du produit de l'exemple 6

| **Doses** μg/kg i.v. | 0 | 0.25 | 0.5 | 1 | **WAY 100,635** 31 μg/kg |
|---|---|---|---|---|---|
| Exemple 6 | 98,11 ± 2,21 | 73,84 ± 14,14 | 52,23 ± 19,38 | 36,16 ± 2,52 | 140,48 ± 20,11 |
| Valeurs individuelles (n = 3). Moyenne ± erreur standard à la moyenne. | | | | | |

L'$ID_{50}$ du composé de l'exemple 6 dans ce test est de 0,59 μg/kg lorsque le produit est administré par voie i. v.. Le résultat démontre que le site d'action, in vivo, des produits de l'invention se situe bien au niveau des récepteurs 5-$HT_{1A}$ comme cela a déjà été démontré in vitro, et que le produit de l'exemple 6 se comporte comme un agoniste sérotoninergique 5-$HT_{1A}$ au niveau pré-synaptique.

2. Test de la température corporelle chez le rat (récepteurs 5-$HT_{1A}$ post-synaptiques)

• Principe

L'administration d'un agoniste sérotoninergique 5-$HT_{1A}$ comme par exemple le 8-OH-DPAT provoque de façon dose-dépendante une diminution de la température corporelle. Cet effet est réversé par les antagonistes 5-$HT_{1A}$ comme l'a démontré M.J. Millan (*J. Pharmacol. Exp. Ther.*, **1993,** 264, 1346-1376). Selon le protocole décrit par cet auteur, les produits de l'invention administrés seuls et qui provoquent une diminution de la température corporelle seront identifiés comme des agonistes sérotoninergiques 5-$HT_{1A}$ post-synaptiques. Par contre, les produits de l'invention qui réversent l'hypothermie induite par l'agoniste prototypique 5-$HT_{1A}$, 8-OH-DPAT, seront identifiés comme des antagonistes 5-$HT_{1A}$ post-synaptiques.

• Résultats

A titre d'exemple et pour illustrer les effets des produits de l'invention, le produit de l'exemple 6 réverse les effets du 8-OH-DPAT avec une $ID_{50}$ de 1,3 mg/kg s.c. Il se comporte donc comme un antagoniste sérotoninergique $5\text{-}HT_{1A}$ post-synaptique

3. Test de vocalisations ultrasoniques chez le rat

• Principe

Lorsqu'un rat est placé dans un environnement associé préalablement à une expérience désagréable (chocs électriques sur les pattes), son anxiété se traduit par l'émission de cris non audibles (ou vocalisations ultrasoniques). L'activité anxiolytique d'un produit se manifeste par une réduction de la durée de ces vocalisations.

• Appareillage

Des boîtes standard (Coulbourn Instruments), placées dans des caissons insonorisants et ventilés, sont équipées d'un sol constitué de barreaux métalliques électrifiables (générateur de chocs et scrambler Med Associates Inc) et d'un microphone situé au centre du plafond. Les ultrasons sont transformés dans une gamme audible (bat-detector Buitenbedrijf). Les signaux ainsi modifiés sont filtrés puis traités (logiciel RTS, Engineering Design). Les spectrogrammes obtenus sont enregistrés sur bandes DAT.

• Méthode

Des rats mâles de souche Wistar pesant 180-200 g à leur arrivée sont logés par cage de quatre avec libre accès à l'eau et à la nourriture, de cinq jours avant le début de l'étude jusqu'à la fin de celle-ci. La procédure employée se décompose en trois étapes successives séparées par 24h, appelées entraînement, sélection et test. Durant la séance d'entraînement, les animaux sont placés individuellement dans les boîtes et y reçoivent six chocs électriques (0,8 mA, 8 s) distribués aléatoirement sur une période de sept minutes. La sélection consiste à placer chaque animal dans une boîte pendant deux minutes pour y recevoir un seul choc, et à l'y replacer trente minutes plus tard pour une séance d'enregistrement des vocalisations ultrasoniques de dix minutes ; les animaux dont la durée des vocalisations est inférieure à 90 secondes sont écartés de la suite de l'expérience. La phase de test se déroule de manière similaire à l'étape de sélection, les produits ou le véhicule étant en outre administrés à la fin de la séance de deux minutes.

• Résultats

A titre d'exemple, le tableau suivant montre les effets du produit de l'exemple 6 administré par voie s.c. sous un volume de 1 ml/kg

| **dose** mg/kg s.c. | **durée des vocalisations ultrasoniques** (s) moyenne ± e.s.m. (n) |
|---|---|
| | Exemple 6 |
| 0 | 230 ± 17 (8) |
| 0,04 | 170 ± 62 (6) |
| 0,16 | 28 ± 14 (3) ** |
| 2,5 | 8 ± 8 (5) ** |

e.s.m. : erreur standard à la moyenne
n : nombre de rats
comparaison versus véhicule (test de Dunnett) : ** $p < 0,01$

Aux doses de 0,16 et 2,5 mg/kg, ce produit entraîne une diminution de la durée des vocalisations, ce qui traduit son activité anxiolytique.

## Revendications

**1.** Les composés de l'indanol de formule I :

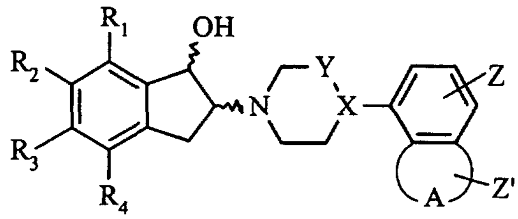

(I)

dans laquelle :

- ♦ $R_1$, $R_2$, $R_3$ et $R_4$ identiques ou différents, représentent chacun un atome d'hydrogène ou d'halogène, un radical $(C_1\text{-}C_5)$alkyle, $(C_2\text{-}C_5)$alkényle ou $(C_2\text{-}C_5)$ alkynyle, chacun en chaîne linéaire ou ramifiée, un radical cycloalkylalkyle dans lequel la partie cycloalkyle renferme de 3 à 7 atomes de carbone et la partie alkyle de 1 à 5 atomes de carbone, un radical trifluorométhyle, CHO, COOH, $COO(C_1\text{-}C_5)$alkyle, $CO(C_1\text{-}C_5)$alkyle, $CH_2OH$, hydroxy, $(C_1\text{-}C_5)$alkoxy, $(C_2\text{-}C_5)$ alkénoxy, $(C_2\text{-}C_5)$alkynoxy, benzyloxy, cyano, nitro,

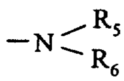 ou 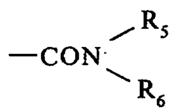

dans lesquels $R_5$ et $R_6$ identiques ou différents représentent chacun un atome d'hydrogène ou un radical $(C_1\text{-}C_5)$alkyle, $\text{-}CO(C_1\text{-}C_5)$alkyle ou $\text{-}COO(C_1\text{-}C_5)$alkyle ; ou

- ♦ $R_1$, $R_2$, $R_3$ et $R_4$ pris deux à deux en position adjacente, forment avec les atomes de carbone du noyau phényle auxquels ils sont liés un cycle de 5 à 7 chaînons, renfermant une ou plusieurs double liaisons et composé d'atomes choisis parmi les atomes de carbone, oxygène, azote et soufre, et les groupements restants, n'étant pas pris deux à deux en position adjacente, représentent alors un atome d'hydrogène,

- ♦ X-Y représente :
$N\text{-}CH_2$, C=CH, $CH\text{-}CH_2$ ou

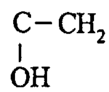 ;

- ♦ A forme avec les deux atomes de carbone du cycle phényle auxquels ils est lié un hétérocycle de 5 à 7 chaînons renfermant une ou plusieurs double liaisons et contenant un ou deux hétéroatomes, identiques ou différents, choisis parmi les atomes d'oxygène et soufre ;

- ♦ Z représente un atome d'hydrogène ou d'halogène, ou un radical hydroxy, $(C_1\text{-}C_5)$alkoxy, et

- ♦ Z' représente un atome d'hydrogène ou un radical oxo, hydroxy, $(C_1\text{-}C_5)$alkoxy ou $CH_2OH$,

sous forme d'isomères cis ou trans, chacun d'eux sous forme racémique ou optiquement active, et leurs sels d'addition avec un acide pharmaceutiquement acceptable.

**2.** Les composés de la revendication 1 répondant à la formule I (identifié dans la revendication 1) dans laquelle :

$R_1$, $R_2$, $R_3$, $R_4$ et X-Y sont tels que définis dans la revendication 1 et

le groupe

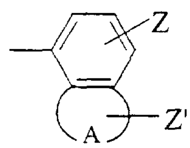

représente plus spécifiquement :

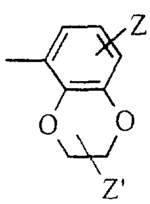, 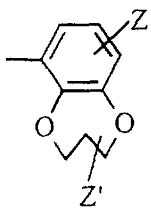, 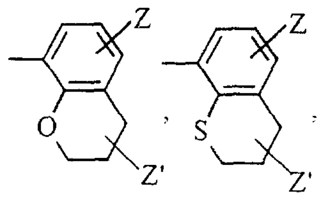, 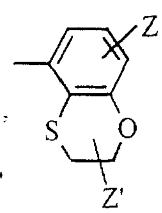, 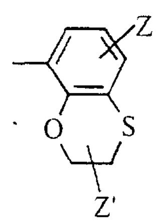 ou 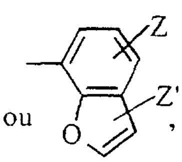,

dans lesquels Z et Z' ont les significations définies, dans la revendication 1, sous forme d'isomères cis ou trans, chacun d'eux sous forme racémique ou optiquement active, et leurs sels d'addition avec un acide pharmaceutiquement acceptable.

3. Un composé de la revendication 1 qui est le trans-2-[4-(2,3-dihydro[1,4]benzodioxin-5-yl)pipérazin-1-yl]-6-méthoxyindan-1-ol.

4. Un composé de la revendication 1 qui est le trans-2-[4-(2,3-dihydro[1,4]benzodioxin-5-yl)pipérazin-1-yl]-5-méthoxyindan-1-ol.

5. Un composé de la revendication 1 qui est le trans-2-[4-(2,3-dihydro[1,4]benzodioxin-5-yl)pipérazin-1-yl]indan-1-ol.

6. Un composé de la revendication 1 qui est le (+)-trans-2-[4-(2,3-dihydro[1,4]benzodioxin-5-yl)pipérazin-1-yl]indan-1-ol.

7. Un composé de la revendication 1 qui est le (-)-trans-2-[4-(2,3-dihydro[1,4]benzodioxin-5-yl)pipérazin-1-yl]indan-1-ol.

8. Un composé de la revendication 1 qui est le trans-2-[4-(2,3-dihydro[1,4]benzodioxin-5-yl)pipérazin-1-yl]-5-fluoroindan-1-ol.

9. Un composé de la revendication 1 qui est le trans-2-[4-(2,3-dihydro[1,4]benzodioxin-5-yl)pipérid-1-yl]-5-fluoroindan-1-ol.

10. Un composé de la revendication 1 qui est le trans-2-[4-(3,4-dihydro-*2H*-chromén-8-yl)pipérazin-1-yl]-5-fluoroindan-1-ol.

11. Un composé de la revendication I qui est le trans-2-{4-[2,3-dihydro-2-(hydroxyméthyl)[1,4]-benzodioxin-5-yl]pipérazin-1-yl}-6-méthoxyindan-1-ol.

12. Un composé de la revendication 1 qui est le trans-2-[4-(2,3-dihydro[1,4]benzodioxin-5-yl)-1,2,3,6-tétrahydropyrid-1-y]-5-fluoroindan-1-ol.

13. Un composé de la revendication 1 qui est le trans-2-[4-(3,4-dihydro-*2H*-thiochromén-8-yl)-1,2,3,6-tétrahydropyrid-1-yl]-5-fluoroindan-1-ol.

14. Un composé de la revendication 1 qui est le trans-2-[4-(3,4-dihydro-*2H*-chromén-8-yl)-1,2,3,6-tetrahydropyrid-1-yl]-5-fluoroindan-1-ol.

**15.** Un composé de la revendication 1 qui est le trans-2-[4-(2,3-dihydro[1,4]benzodioxin-5-yl)pipérid-1-yl]indan-1-ol

**16.** Un composé de la revendication 1 qui est le trans-2-[4-(benzofuran-7-yl)pipérid-1-yl]-5-fluoroindan-1-ol.

**17.** Un composé de la revendication 1 qui est le trans-2-[4-(2,3-dihydro[1,4]benzodioxin-5-yl)pipérid-1-yl]-6-fluoroindan-1-ol.

**18.** Un composé de la revendication 1 qui est le trans-2-[4-(2,3-dihydro[1,4]benzodioxin-5-yl)pipérid-1-yl]-5-méthylindan-1-ol.

**19.** Un composé de la revendication 1 qui est le trans-2-[4-(2,3-dihydro[1,4]benzodioxin-5-yl)pipérid-1-yl]-6-méthylindan-1-ol.

**20.** Un composé de la revendication 1 qui est le trans-2-[4-(3,4-dihydro-6-fluoro-*2H*-chromén-8-yl)pipérid-1-yl]-5-fluoroindan-1-ol.

**21.** Un composé de la revendication 1 qui est le trans-2- [4-(2,3-dihydro[1,4]benzodioxin-5-yl)pipérid-1-yl]-5,6-méthylènedioxyindan-1-ol.

**22.** Un composé de la revendication 1 qui est le trans-2-[4-(2,3-dihydrobenzofuran-7-yl)pipérid-1-yl]-5-fluoroindan-1-ol.

**23.** Un composé de la revendication 1 qui est le trans-2-[4-(2,3-dihydro[1,4]benzodioxin-5-yl)-4-hydroxypipérid-1-yl]-5-fluoroindan-1-ol.

**24.** Un composé de la revendication 1 qui est le trans-2-[4-(7-chloro-2,3-dihydro[1,4]benzodioxin-5-yl)pipérid-1-yl]-5-fluoroindan-1-ol.

**25.** Un composé de la revendication 1 qui est le trans-2-[4-(3,4-dihydro-*2H*-chromén-8-yl)pipérid-1-yl]-5-fluoroindan-1-ol.

**26.** Un composé de la revendication 1 qui est le trans-2-[4-(2,3-dihydro[1,4]benzodioxin-5-yl)pipérid-1-yl]-6-trifluorométhylindan-1-ol.

**27.** Un composé de la revendication 1 qui est le trans-2-[4-(3,4-dihydro[1,4]-*2H*-chromén-8-yl)pipérid-1-yl]indan-1-ol.

**28.** Un composé de la revendication 1 qui est le trans-2-{4-[2,3-dihydro-2-(hydroxyméthyl)[1,4]benzodioxin-5-yl]pipérid-1-yl}-5-fluoroindan-1-ol.

**29.** Un composé de la revendication 1 qui est le trans-2-{4-[(3,4-dihydro)-4-oxo-*2H*-chromén-8-yl]pipérid-1-yl}-5-fluoroindan-1-ol.

**30.** Un composé de la revendication 1 qui est le trans-2-{4-[(3,4-dihydro)-4-hydroxy-*2H*-chromén-8-yl]pipérid-1-yl}-5-fluoroindan-1-ol.

**31.** Le procédé de préparation des composés de la revendication 1 **caractérisé en ce que** :

- une cétone de formule II :

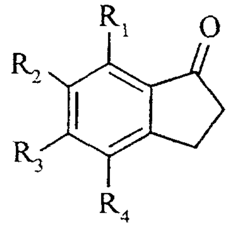

(II)

dans laquelle $R_1$, $R_2$, $R_3$ et $R_4$ ont les significations définies à la revendication 1,
est halogénée en position $\alpha$ de la fonction cétone pour obtenir un composé de formule III :

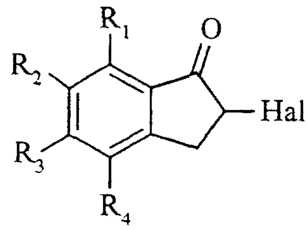

(III)

dans laquelle $R_1$, $R_2$, $R_3$, $R_4$ prennent les valeurs précédemment définies et Hal représente un atome de chlore, brome ou iode,
lequel composé III est mis à réagir avec une amine de formule IV :

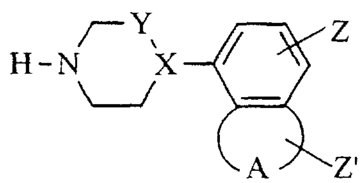

(IV)

dans laquelle X-Y, A, Z et Z' ont les significations définies à la revendication 1,
pour donner un composé de formule V :

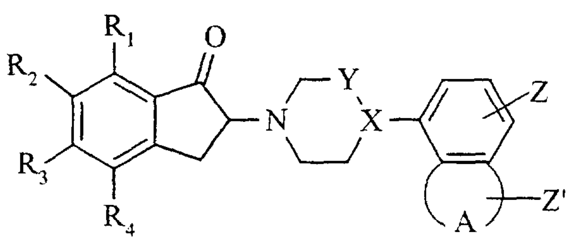

(V)

dans laquelle $R_1$, $R_2$, $R_3$, $R_4$, X-Y, A, Z et Z' ont les significations précédemment définies
qui est réduit en un mélange d'amino-alcools de formules I trans et I cis :

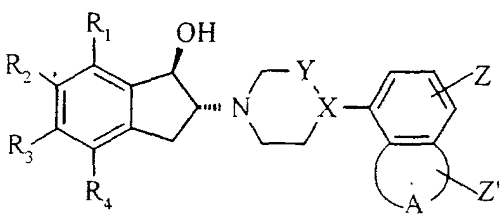

(I trans)

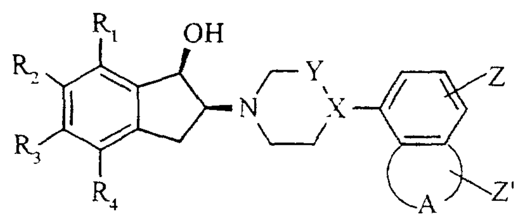

(I cis)

dans lesquelles $R_1$, $R_2$, $R_3$, $R_4$, X-Y, A Z et Z' ont les significations précédemment définies,
les composés I trans et I cis sont ensuite séparés par les méthodes classiques de séparation, et, si on le désire,

- on dédouble les formes racémiques pour obtenir les formes optiquement actives correspondantes, et/ou
- on salifie les composés I obtenus avec des acides pharmaceutiquement acceptables pour obtenir les sels correspondants.

**32.** Le procédé de préparation des composés de formule I, selon la revendication 1, dans laquelle X-Y représente C=CH ou CH-$CH_2$, c'est-à-dire les composés répondant plus spécifiquement à la formule Ia :

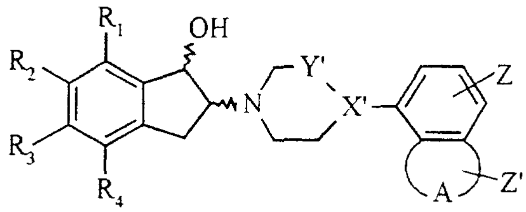

(Ia)

dans laquelle :

$R_1$, $R_2$, $R_3$, $R_4$, A, Z et Z' ont les significations définies à la revendication 1 et X'-Y' représente C=CH ou CH-$CH_2$,

**caractérisé en ce que** l'on fait réagir un composé de formule VI :

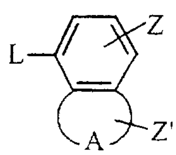

(VI)

dans laquelle

A, Z et Z' ont les significations définies à la revendication 1, et L représente un groupe labile choisi parmi Br, I ou $OSO_2CF_3$,

- avec le diéthyl(pyrid-1-yl)borane dans les conditions de la réaction de Suzuki,
- pour obtenir un composé de formule VII :

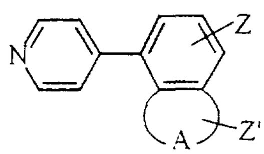

(VII)

dans laquelle A, Z et Z' ont les significations précédemment définies,

- que l'on fait réagir avec un composé de formule III définie à la revendication 31, pour obtenir un composé de formule VIII :

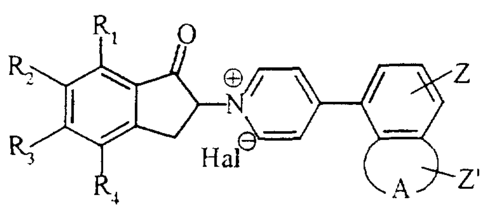

(VIII)

dans laquelle $R_1$, $R_2$, $R_3$, $R_4$, A, Z, Z' et Hal ont les significations définies à la revendication 31,
- que l'on réduit par un borohydrure métallique dans un solvant alcoolique pour conduire à un mélange d'amino-alcools de formule I'a trans et I'a cis :

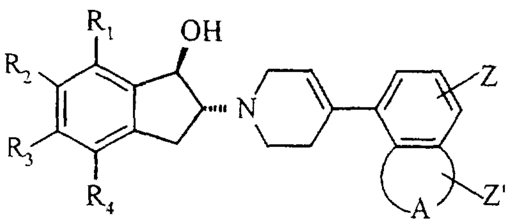

(I'a trans)

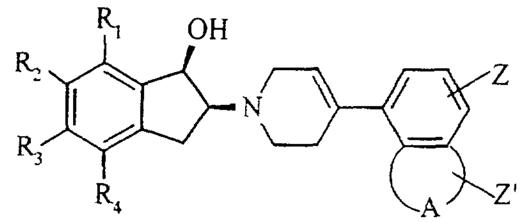

(I'a cis)

dans lesquelles $R_1$, $R_2$, $R_3$, $R_4$, A, Z et Z' ont les significations précédemment définies,

- lesquels aminoalcools sont, si on le désire, réduits à nouveau, par de l'hydrogène en présence d'un catalyseur approprié, en composés de formule I"a trans et I"a cis :

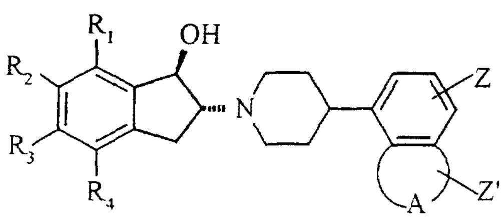

(I"a trans)

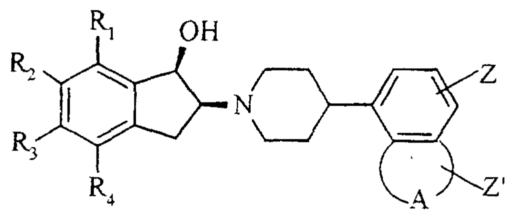

(I"a cis)

dans lesquelles $R_1$, $R_2$, $R_3$, $R_4$, A, Z et Z' ont les significations précédemment définies,

- et dans le cas où Z' prend la valeur oxo, il est judicieux de partir d'un composé VII dans lequel cette fonction oxo est d'abord protégée, puis déprotégée soit au niveau des composés I'a trans et I'a cis soit au niveau des composés I"a trans et I"a cis,
- les composés I'a trans et I'a cis d'une part et I"a trans et I"a cis d'autre part, sont ensuite séparés par les méthodes classiques de séparation des composés organiques,

et, si on le désire :

- on dédouble les formes racémiques pour obtenir les formes optiquement actives correspondantes, et/ou,
- on salifie les composés la obtenus avec des acides pharmaceutiquement acceptables pour obtenir les sels correspondants.

**33.** Compositions pharmaceutiques contenant comme principe actif au moins un composé selon l'une quelconque des revendications 1 à 30, mélangé ou associé avec un ou plusieurs excipients ou véhicules inertes, non toxiques, pharmaceutiquement acceptables.

**34.** Compositions pharmaceutiques selon la revendication 33 contenant au moins un principe actif selon l'une quelconque des revendications 1 à 30, utiles dans le traitement des maladies du système nerveux central ou des manifestations de la douleur.

**Patentansprüche**

**1.** Indanol-Verbindungen der Formel I:

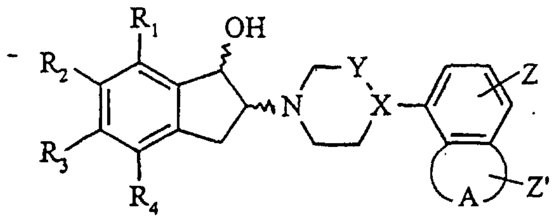

(I)

in, der:

- ♦ $R_1$, $R_2$, $R_3$ und $R_4$, die gleichartig oder verschieden sind, jeweils ein Wasserstoffatom oder ein Halogenatom, eine ($C_1$-$C_5$)-Alkylgruppe, ($C_2$-$C_5$)-Alkenylgruppe oder ($C_1$-$C_5$)-Alkinylgruppe mit jeweils gerader oder verzweigter Kette, eine Cycloalkylalkylgruppe, in der der Cycloalkylrest 3 bis 7 Kohlenstoffatome und der Alkylrest 1 bis 5 Kohlenstoffatome aufweist, eine Trifluormethylgruppe, CHO, COOH, COO($C_1$-$C_5$)-Alkyl, CO($C_1$-$C_5$)-Alkyl, $CH_2OH$, Hydroxy, ($C_1$-$C_5$)-Alkoxy, ($C_2$-$C_5$)-Alkenoxy, ($C_2$-$C_5$)-Alkinoxy, Benzyloxy, Cyano, Nitro,

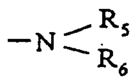 oder 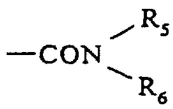

worin $R_5$ und $R_6$, die gleichartig oder verschieden sind, jeweils ein Wasserstoffatom oder eine ($C_1$-$C_5$)-Alkylgruppe, -CO($C_1$-$C_5$)-Alkylgruppe oder -COO($C_1$-$C_5$)-Alkylgruppe darstellen, bedeuten; oder
- ♦ $R_1$, $R_2$, $R_3$ und $R_4$ jeweils in Gruppen von zwei in benachbarter Position zusammen mit den Kohlenstoffatomen des Phenylkerns, an die sie gebunden sind, einen Ring mit 5 bis 7 Kettengliedern bilden, der eine oder mehrere Doppelbindungen aufweist und aus Atomen gebildet ist, die aus Kohlenstoff-, Sauerstoff-, Stickstoff- und Schwefelatomen ausgewählt sind, und die verbleibenden Gruppen, die nicht Zweiergruppen in benachbarter Position darstellen, Wasserstoffatome bedeuten;
- ♦ X - Y:
  N-$CH_2$, C=CH, CH-$CH_2$ oder

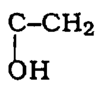 ;

darstellen;
- ♦ A mit den beiden Kohlenstoffatomen des Phenylrings, an die es gebunden ist, einen Heterocyclus mit 5 bis 7 Kettengliedern bildet, der eine oder mehrere Doppelbindungen aufweist und ein oder zwei gleichartige oder verschiedene Heteroatome ausgewählt aus Sauerstoff- und Schwefelatomen enthält;
- ♦ Z ein Wasserstoff- oder Halogenatom oder eine Hydroxygruppe oder ($C_1$-$C_5$)-Alkoxygruppe bedeutet und
- ♦ Z' ein Wasserstoffatom oder eine Oxo-, Hydroxy-, ($C_1$-$C_5$)-Alkoxy- oder $CH_2OH$-Gruppe darstellt,

in Form der cis- oder trans-Isomeren, wobei die Verbindungen jeweils in Form des Racemats oder in optisch aktiver Form vorliegen, und deren Additionssalze mit einer pharmazeutisch annehmbaren Säure.

**2.** Verbindungen nach Anspruch 1 der Formel I (wie sie in Anspruch 1 definiert ist), in der:

$R_1$, $R_2$, $R_3$, $R_4$ und X-Y die in Anspruch 1 angegebenen Bedeutungen besitzen und die Gruppe

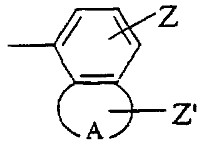

insbesondere:

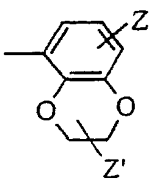 , 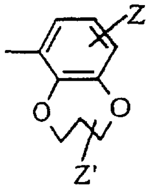 ; 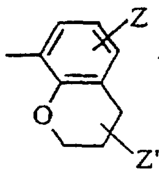 , 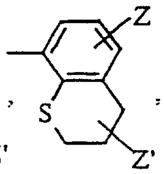 ; 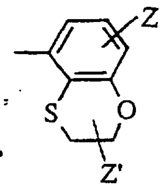 . 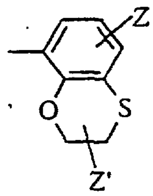 oder 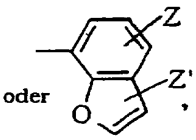 ,

darstellt, worin Z und Z' die in Anspruch 1 angegebenen Bedeutungen besitzen, in Form der cis- oder trans-Isomeren, jeweils in racemischer Form oder optisch aktiver Form vorliegen können, und deren Additionssalze mit einer pharmazeutisch annehmbaren Säure.

**3.** Verbindung nach Anspruch 1, nämlich trans-2-[4-(2,3-Dihydro[1,4]benzodioxin-5-yl)-piperazin-1-yl]-6-methoxyindan-1-ol.

**4.** Verbindung nach Anspruch 1, nämlich trans-2-[4-(2,3-Dihydro[1,4]benzodioxin-5-yl)-piperazin-1-yl]-5-methoxyindan-1-ol.

**5.** Verbindung nach Anspruch 1, nämlich trans-2-[4-(2,3-Dihydro[1,4]benzodioxin-5-yl)-piperazin-1-yl]-indan-1-ol.

**6.** Verbindung nach Anspruch 1, nämlich (+)-trans-2-[4-(2,3-Dihydro[1,4]benzodioxin-5-yl)-piperazin-1-yl]-indan-1-ol.

**7.** Verbindung nach Anspruch 1, nämlich (-)-trans-2-[4-(2,3-Dihydro[1,4]benzodioxin-5-yl)-piperazin-1-yl]-indan-1-ol.

**8.** Verbindung nach Anspruch 1, nämlich trans-2-[4-(2,3-Dihydro[1,4]benzodioxin-5-yl)-piperazin-1-yl]-5-fluorindan-1-ol.

**9.** Verbindung nach Anspruch 1, nämlich trans-2-[4-(2,3-Dihydro[1,4]benzodioxin-5-yl)-piperid-1-yl]-5-fluorindan-1-ol.

**10.** Verbindung nach Anspruch 1, nämlich trans-2-[4-(3,4-Dihydro-*2H*-chromen-8-yl)-piperazin-1-yl]-5-fluorindan-1-ol.

**11.** Verbindung nach Anspruch 1, nämlich trans-2-{4-[2,3-Dihydro-2-(hydroxymethyl)-[1,4]-benzodioxin-5-yl)-piperazin-1-yl}-6-methoxyindan-1-ol.

**12.** Verbindung nach Anspruch 1, nämlich trans-2-[4-(2,3-Dihydro[1,4]benzodioxin-5-yl)-1,2,3,6-tetrahydropyrid-1-yl]-5-fluorindan-1-ol.

**13.** Verbindung nach Anspruch 1, nämlich trans-2-[4-(3,4-Dihydro-*2H*-thiochromen-8-yl)-1,2,3,6-tetrahydropyrid-1-yl]-5-fluorindan-1-ol.

**14.** Verbindung nach Anspruch 1, nämlich trans-2-[4-(3,4-Dihydro-*2H*-chromen-8-yl)-1,2,3, 6-tetrahydropyrid-1-yl]-5-fluorindan-1-ol.

**15.** Verbindung nach Anspruch 1, nämlich trans-2-[4-(2,3-Dihydro[1,4]benzodioxin-5-yl)-piperid-1-yl]-indan-1-ol.

**16.** Verbindung nach Anspruch 1, nämlich trans-2-[4-(Benzofuran-7-yl)-piperid-1-yl]-5-fluorindan-1-ol.

**17.** Verbindung nach Anspruch 1, nämlich trans-2-[4-(2,3-Dihydro[1,4]benzodioxin-5-yl)-piperid-1-yl]-6-fluorindan-1-ol.

**18.** Verbindung nach Anspruch 1, nämlich trans-2-[4-(2,3-Dihydro[1,4]benzodioxin-5-yl)-piperid-1-yl]-5-methylindan-1-ol.

**19.** Verbindung nach Anspruch 1, nämlich trans-2-[4-(2,3-Dihydro[1,4]benzodioxin-5-yl)-piperid-1-yl]-6-methylindan-1-ol.

**20.** Verbindung nach Anspruch 1, nämlich trans-2-[4-(3,4-Dihydro-6-fluor-*2H*-chromen-8-yl)-piperid-1-yl]-5-fluorindan-1-ol.

**21.** Verbindung nach Anspruch 1, nämlich trans-2-[4-(2,3-Dihydro[1,4]benzodioxin-5-yl)-piperid-1-yl]-5,6-methylendioxyindan-1-ol.

**22.** Verbindung nach Anspruch 1, nämlich trans-2-[4-(2,3-Dihydrobenzofuran-7-yl)-piperid-1-yl]-5-fluorindan-1-ol.

**23.** Verbindung nach Anspruch 1, nämlich trans-2-[4-(2,3-Dihydro[1,4]benzodioxin-5-yl)-4-hydroxypiperid-1-yl]-5-fluorindan-1-ol.

**24.** Verbindung nach Anspruch 1, nämlich trans-2-[4-(7-Chlor-2,3-dihydro[1,4]benzodioxin-5-yl)-piperid-1-yl]-5-fluorindan-1-ol.

**25.** Verbindung nach Anspruch 1, nämlich trans-2-[4-(3,4-Dihydro-*2H*-chromen-8-yl)-piperid-1-yl]-5-fluorindan-1-ol.

**26.** Verbindung nach Anspruch 1, nämlich trans-2-[4-(2,3-Dihydro[1,4]benzodioxin-5-yl)-piperid-1-yl]-6-trifluormethylindan-1-ol.

**27.** Verbindung nach Anspruch 1, nämlich trans-2-[4-(3,4-Dihydro[1,4]-*2H*chromen-8-yl)-piperid-1-yl]-indan-1-ol.

**28.** Verbindung nach Anspruch 1, nämlich trans-2-{4-[2,3-Dihydro-2-(hydroxymethyl)[1,4]benzodioxin-5-yl)-piperid-1-yl}-5-fluorindan-1-ol.

**29.** Verbindung nach Anspruch 1, nämlich trans-2-{4-[(3,4-Dihydro)-4-oxo-*2H*chromen-8-yl)-piperid-1-yl}-5-fluorindan-1-ol.

**30.** Verbindung nach Anspruch 1, nämlich trans-2-{4-[(3,4-Dihydro)-4-hydroxy-*2H*-chromen-8-yl)-piperid-1-yl}-5-fluorindan-1-ol.

**31.** Verfahren zur Herstellung der Verbindungen nach Anspruch 1, **dadurch gekennzeichnet, daß** man:

- ein Keton der Formel II:

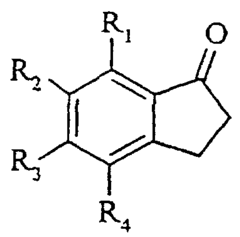

(II)

in der $R_1$, $R_2$, $R_3$ und $R_4$ die in Anspruch 1 angegebenen Bedeutungen besitzen, in der α-Stellung der Keto-Funktion halogeniert zur Bildung einer Verbindung der Formel III:

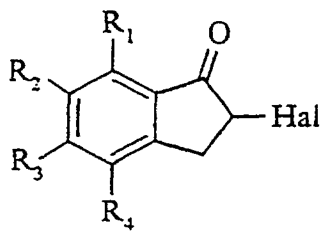

(III)

in der $R_1$, $R_2$, $R_3$ und $R_4$ die oben angegebenen Bedeutungen besitzen und Hal ein Chlor-, Brom- oder Iodatom bedeutet,
welche Verbindung III mit einem Amin der Formel IV:

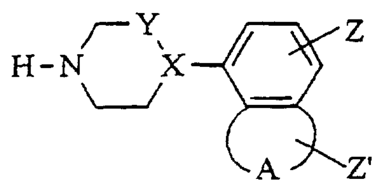

(IV)

in der X-Y, A, Z und Z' die in Anspruch 1 angegebenen Bedeutungen besitzen, umgesetzt wird
zur Bildung einer Verbindung der Formel V:

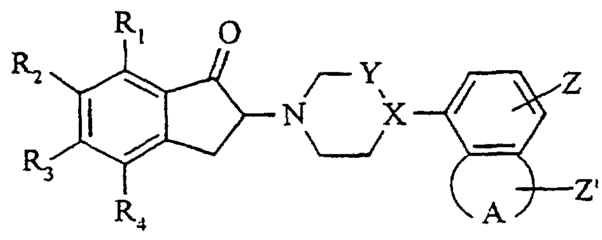

(V)

in der $R_1$, $N_2$, $R_3$, $R_4$, X-Y, A, Z und Z' die oben angegebenen Bedeutungen besitzen,
welche zu einer Mischung von Aminoalkoholen der Formeln I trans und I cis reduziert wird:

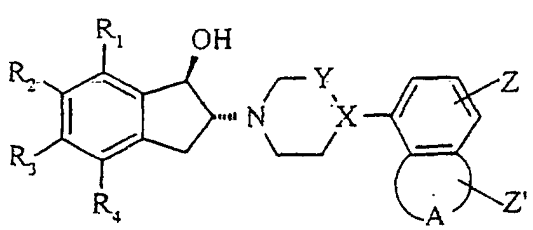

(I trans)

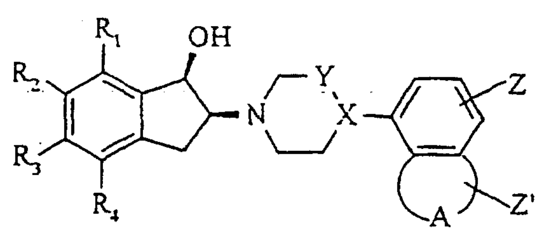

(I cis)

in denen $R_1$, $R_2$, $R_3$, $R_4$, X-Y, A, Z und Z' die oben angegebenen Bedeutungen besitzen,
welche Verbindungen I trans und I cis anschließend mit Hilfe klassischer Trennmethoden getrennt werden und gewünschtenfalls

- man die racemischen Formen aufspaltet zur Bildung der entsprechenden optisch aktiven Formen und/oder
- man die erhaltenen Verbindungen I mit pharmazeutisch annehmbaren Säuren in die entsprechenden Salze umwandelt.

**32.** Verfahren zur Herstellung der Verbindungen der Formel I nach Anspruch 1, worin X-Y C=CH oder $CH-CH_2$ bedeutet, das heißt der Verbindungen, die genauer der Formel Ia entsprechen:

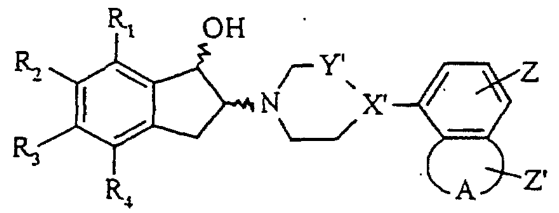

(Ia)

in der:
$R_1$, $R_2$, $R_3$, $R_4$, A, Z und Z' die in Anspruch 1 angegebenen Bedeutungen besitzen und X'-Y' C=CH oder $CH-CH_2$ bedeuten,
**dadurch gekennzeichnet, daß** man eine Verbindung der Formel VI:

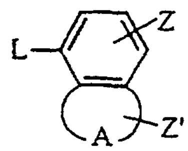

(VI)

in der:

A, Z und Z' die in Anspruch 1 angegebenen Bedeutungen besitzen und
L eine labile Gruppe ausgewählt aus Br, I und $OSO_2CF_3$ darstellt,

- mit Diethyl-(pyrid-1-yl)-boran unter den Bedingungen der Suzuki-Reaktion umsetzt,
- zur Bildung einer Verbindung der Formel VII:

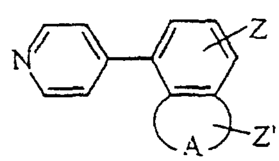

(VII)

in der A, Z und Z' die oben angegebenen Bedeutungen besitzen.
- welche man mit einer Verbindung der Formel III, wie sie in Anspruch 31 definiert ist, umsetzt zur Bildung einer Verbindung der Formel VIII:

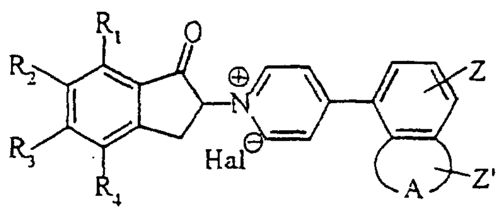

(VIII)

in der $R_1$, $R_2$, $R_3$, $R_4$, A, Z, Z' und Hal die in Anspruch 31 angegebenen Bedeutungen besitzen,
- welche man mit einem Metallborhydrid in einem alkoholischen Lösungsmittel reduziert zur Bildung einer Mischung von Aminoalkoholen der Formel I'a trans und I'a cis:

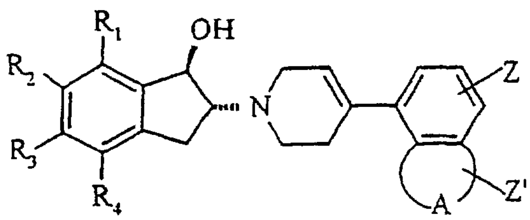

(I'a trans)

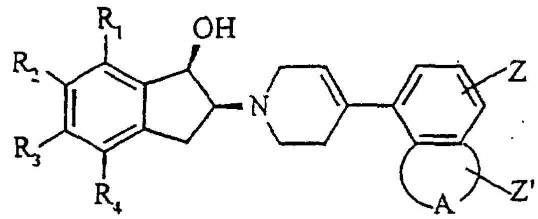

(I'a cis)

in denen $R_1$, $R_2$, $R_3$, $R_4$, A, Z und Z' die oben angegebenen Bedeutungen besitzen,

- welche Aminoalkohole gewünschtenfalls erneut mit Wasserstoff in Gegenwart eines geeigneten Katalysators zu den Verbindungen der Formeln I"a trans und I"a cis reduziert werden:

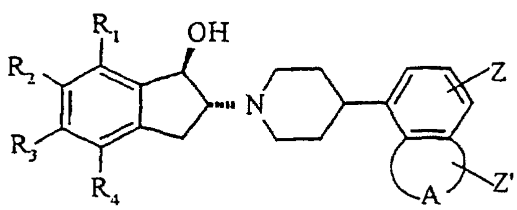

(I"a trans)

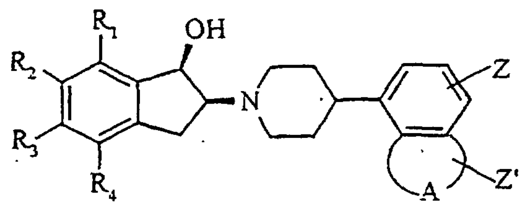

(I"a cis)

in denen $R_1$, $R_2$, $R_3$, $R_4$, A, Z und Z' die oben angegebenen Bedeutungen besitzen,

- und dann, wenn Z' die Bedeutung oxo besitzt, es sinnvoll ist, von einer Verbindung der Formel VII auszugehen, in der diese oxo-Funktion zunächst geschützt ist, und diese dann entweder in Form der Verbindungen I'a trans und I'a cis oder in Form der Verbindungen I"a trans und I"a cis von der Schutzgruppe befreit,
- welche Verbindungen I'a trans und I'a cis einerseits und I"a trans und I"a cis andererseits anschließend mit Hilfe klassischer Trennmethoden für organische Verbindungen getrennt werden,

und gewünschtenfalls:

- man die racemischen Formen aufspaltet zur Bildung der entsprechenden optisch aktiven Formen und/oder
- man die erhaltenen Verbindungen Ia mit pharmazeutisch annehmbaren Säuren in die entsprechenden Salze umwandelt.

**33.** Pharmazeutische Zubereitungen enthaltend als Wirkstoff mindestens eine Verbindung nach einem der Ansprüche 1 bis 30 in Mischung oder in Kombination mit einem oder mehreren inerten, nichttoxischen, pharmazeutisch annehmbaren Trägermaterialien oder Bindemitteln.

**34.** Pharmazeutische Zubereitungen nach Anspruch 33 enthaltend mindestens einen Wirkstoff nach einem der Ansprüche 1 bis 30 für die Behandlung von Erkrankungen des Zentralnervensystems oder von Schmerzmanifestationen.

## Claims

**1.** Indanol compounds of formula I:

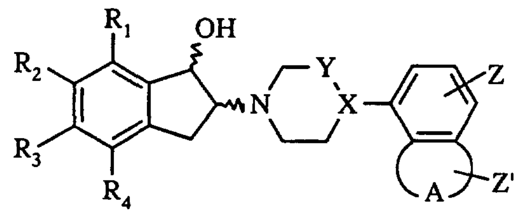

(I)

wherein :

- $R_1$, $R_2$, $R_3$ and $R_4$, which may be identical or different, each represent a hydrogen or halogen atom, a $(C_1\text{-}C_5)$ alkyl, $(C_2\text{-}C_5)$alkenyl or $(C_2\text{-}C_5)$alkynyl radical, those radicals being linear or branched chain, a cycloalkylalkyl radical in which the cycloalkyl moiety contains from 3 to 7 carbon atoms and the alkyl moiety contains from 1 to 5 carbon atoms, a trifluoromethyl, CHO, COOH, $COO(C_1\text{-}C_5)$alkyl, $CO(C_1\text{-}C_5)$alkyl, $CH_2OH$, hydroxy, $(C_1\text{-}C_5)$alkoxy, $(C_2\text{-}C_5)$alkenoxy, $(C_2\text{-}C_5)$alkynoxy, benzyloxy, cyano or nitro radical,

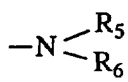 or 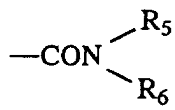

  wherein $R_5$ and $R_6$, which may be identical or different, each represent a hydrogen atom or a $(C_1\text{-}C_5)$alkyl, $-CO(C_1\text{-}C_5)$alkyl or $-COO(C_1\text{-}C_5)$alkyl radical; or

- $R_1$, $R_2$, $R_3$ and $R_4$, taken in pairs in adjacent positions, form with the carbon atoms of the phenyl nucleus to which they are bonded a 5- to 7-membered ring, containing one or more double bonds and composed of atoms selected from carbon, oxygen, nitrogen and sulphur atoms, and the remaining groups, not being taken in pairs in adjacent positions, then represent a hydrogen atom,

- X-Y represents:
  $N\text{-}CH_2$, C=CH, $CH\text{-}CH_2$ or

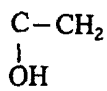 ;

- A forms with the two carbon atoms of the phenyl ring to which it is bonded a 5 to 7-membered heterocycle containing one or more double bonds and comprising one or two hetero atoms, which may be identical or different, selected from oxygen and sulphur atoms;

- Z represents a hydrogen or halogen atom or a hydroxy or $(C_1\text{-}C_5)$alkoxy radical, and

- Z' represents a hydrogen atom or an oxo, hydroxy, $(C_1\text{-}C_5)$alkoxy or $CH_2OH$ radical,

in the form of the *cis* or *trans* isomers, each of those being in racemic or optically active form, and addition salts thereof with a pharmaceutically acceptable acid.

**2.** Compounds of claim 1 corresponding to formula I (identified in claim 1) wherein :

$R_1$, $R_2$, $R_3$, $R_4$ and X-Y are as defined in claim 1 and
the

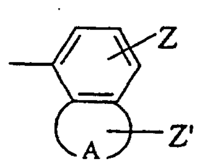

group represents more specifically :

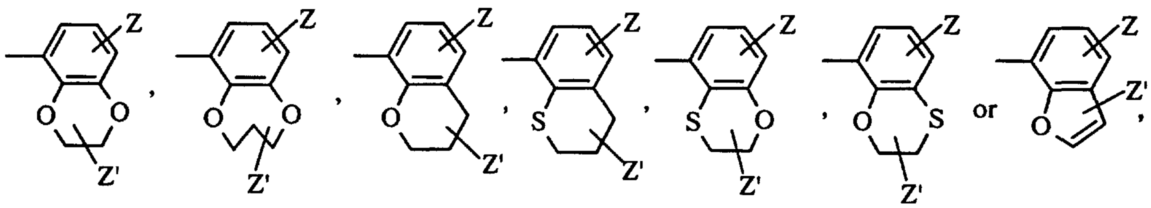

, , , , , or ,

wherein Z and Z' are as defined in claim 1, in the form of the *cis* or *trans* isomers, each of those being in racemic or optically active form, and addition salts thereof with a pharmaceutically acceptable acid.

**3.** A compound of claim 1 which is *trans*-2-[4-(2,3-dihydro[1,4]benzodioxin-5-yl) piperazin-1-yl]-6-methoxyindan-1-ol.

**4.** A compound of claim 1 which is *trans*-2-[4-(2,3-dihydro[1,4]benzodioxin-5-yl)piperazin-1-yl]-5-methoxyindan-1-ol.

**5.** A compound of -claim 1 which is *trans*-2-[4-(2,3-dihydro[1,4]benzodioxin-5-yl)piperazin-1-yl]indan-1-ol.

**6.** A compound of claim 1 which is (+)-*trans*-2-[4-(2,3-dihydro[1,4]benzodioxin-5-yl)piperazin-1-yl]indan-1-ol.

**7.** A compound of claim 1 which is (-)-*trans*-2-[4-(2,3-dihydro[1,4]benzodioxin-5-yl)piperazin-1-yl]indan-1-ol.

**8.** A compound of claim 1 which is *trans*-2-[4-(2,3-dihydro[1,4]benzodioxin-5-yl)piperazin-1-yl]-5-fluoroindan-1-ol.

**9.** A compound of claim 1 which is *trans*-2-[4-(2,3-dihydro[1,4]benzodioxin-5-yl)piperid-1-yl]-5-fluoroindan-1-ol.

**10.** A compound of claim 1 which is *trans*-2-[4-(3,4-dihydro-*2H*-chromen-8-yl)piperazin-1-yl]-5-fluoroindan-1-ol.

**11.** A compound of claim 1 which is *trans*-2-{4-[2,3-dihydro-2-(hydroxymethyl)[1,4]-benzodioxin-5-yl]piperazin-1-yl}-6-methoxyindan-1 -ol.

**12.** A compound of claim 1 which is *trans*-2-[4-(2,3-dihydro[1,4]benzodioxin-5-yl)-1,2,3,6-tetrahydropyrid-1-yl]-5-fluoroindan-1-ol.

**13.** A compound of claim 1 which is *trans*-2-[4-(3,4-dihydro-*2H*-thiochromen-8-yl)-1,2,3,6-tetrahydropyrid-1-yl]-5-fluoroindan-1-ol.

**14.** A compound of claim 1 which is *trans*-2-[4-(3,4-dihydro-*2H*-chromen-8-yl)-1,2,3,6-tetrahydropyrid-1-yl]-5-fluoroindan-1-ol.

**15.** A compound of claim 1 which is *trans*-2-[4-(2,3-dihydro[1,4]benzodioxin-5-yl)piperid-1-yl]indan-1-ol.

**16.** A compound of claim 1 which is *trans*-2-[4-(benzofuran-7-yl)piperid-1-yl]-5-fluoroindan-1-ol.

**17.** A compound of claim 1 which is *trans*-2-[4-(2,3-dihydro[1,4]benzodioxin-5-yl)piperid-1-yl]-6-fluoroindan-1-ol.

**18.** A compound of claim 1 which is *trans*-2-[4-(2,3-dihydro[1,4]benzodioxin-5-yl)piperid-1-yl]-5-methylindan-1-ol.

**19.** A compound of claim 1 which is *trans*-2-[4-(2,3-dihydro[1,4]benzodioxin-5-yl)piperid-1-yl]-6-methylindan-1-ol.

**20.** A compound of claim 1 which is *trans*-2-[4-(3,4-dihydro-6-fluoro-*2H*-chromen-8-yl)piperid-1-yl]-5-fluoroindan-1-ol.

**21.** A compound of claim 1 which is *trans*-2-[4-(2,3-dihydro[1,4]benzodioxin-5-yl)piperid-1-yl]-5,6-methylenedioxyindan-1-ol.

**22.** A compound of claim 1 which is *trans*-2-[4-(2,3-dihydrobenzofuran-7-yl)piperid-1-yl]-5-fluoroindan-1-ol.

**23.** A compound of claim 1 which is *trans*-2-[4-(2,3-dihydro[1,4]benzodioxin-5-yl)-4-hydroxypiperid-1-yl]-5-fluoroindan-1-ol.

**24.** A compound of claim 1 which is *trans*-2-[4-(7-chloro-2,3-dihydro[1,4]benzodioxin-5-yl)piperid-1-yl]-5-fluoroindan-1-ol.

**25.** A compound of claim 1 which is *trans*-2-[4-(3,4-dihydro-*2H*-chromen-8-yl)piperid-1-yl]-5-fluoroindan-1-ol.

**26.** A compound of claim 1 which is *trans*-2-[4-(2,3-dihydro[1,4]benzodioxin-5-yl)piperid-1-yl]-6-trifluoromethylindan-1-ol.

**27.** A compound of claim 1 which is *trans*-2-[4-(3,4-dihydro[1,4]-*2H*-chromen-8-yl)piperid-1-yl]indan-1-ol.

**28.** A compound of claim 1 which is *trans*-2-{4-[2,3-dihydro-2-(hydroxymethyl)-[1,4]benzodioxin-5-yl]piperid-1-yl}-5-fluoroindan-1-ol.

**29.** A compound of claim 1 which is *trans*-2-{4-[(3,4-dihydro)-4-oxo-*2H*-chromen-8-yl]-piperid-1-yl} -5-fluoroindan-1-ol.

**30.** A compound of claim 1 which is *trans*-2-{4-[(3,4-dihydro)-4-hydroxy-*2H*-chromen-8-yl]piperid-1-yl}-5-fluoroindan-1-ol.

**31.** A process for the preparation of compounds of claim 1, **characterised in that**:

- a ketone of formula II:

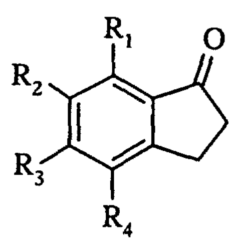

(II)

wherein $R_1$, $R_2$, $R_3$ and $R_4$ are as defined in claim 1,
is halogenated in the position $\alpha$ to the ketone function to obtain a compound of formula III:

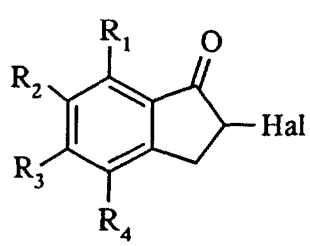

(III)

wherein $R_1$, $R_2$, $R_3$, $R_4$ are as defined hereinbefore and Hal represents a chlorine, bromine or iodine atom, which compound III is reacted with an amine of formula IV :

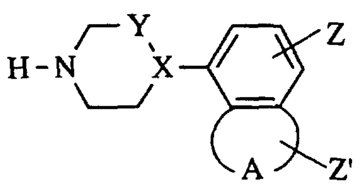

(IV)

wherein X-Y, A, Z and Z' are as defined in claim 1, to yield a compound of formula V:

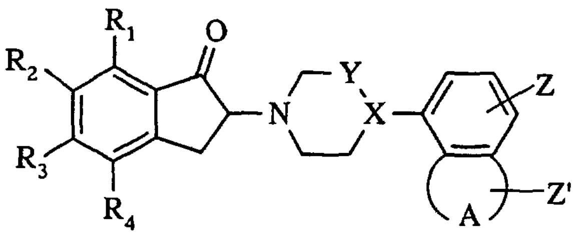

(V)

wherein $R_1$, $R_2$, $R_3$, $R_4$, X-Y, A, Z and Z' are as defined hereinbefore, which is reduced to a mixture of amino alcohols of formulae I *trans* and I *cis* :

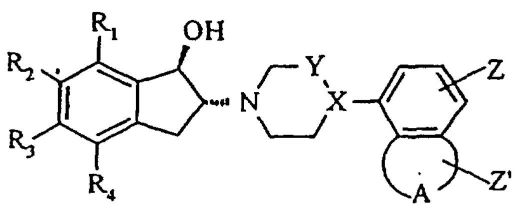

(I trans)

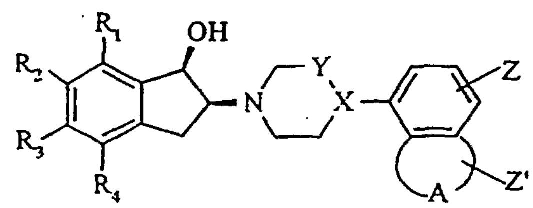

(I cis)

wherein $R_1$, $R_2$, $R_3$, $R_4$, X-Y, A, Z and Z' are as defined hereinbefore, which compounds I *trans* and I *cis* are then separated by conventional separation methods and, if desired,

- the racemic forms are separated to obtain the corresponding optically active forms, and/or
- the resulting compounds I are converted into salts with pharmaceutically acceptable acids to obtain the corresponding salts.

**32.** A process for the preparation of compounds of formula I, according to claim 1, wherein X-Y represents C=CH or $CH\text{-}CH_2$, that is to say compounds corresponding more specifically to formula Ia :

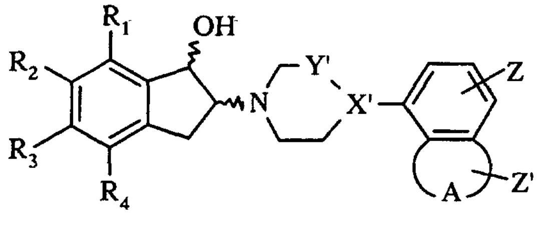

(Ia)

wherein:

$R_1$, $R_2$, $R_3$, $R_4$, A, Z and Z' are as defined in claim 1 and

X'-Y' represents C=CH or $CH-CH_2$,

**characterised in that** a compound of formula VI:

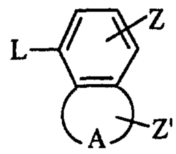

(VI)

wherein:

A, Z and Z' are as defined in claim 1, and
L represents a labile group selected from Br, I and $OSO_2CF_3$, is reacted with

- diethyl(pyrid-1-yl)borane under the conditions of the Suzuki reaction,
- to obtain a compound of formula VII:

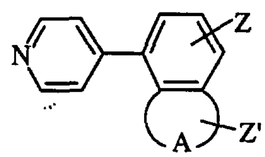

(VII)

wherein A, Z and Z' are as defined hereinbefore,
- which is reacted with a compound of formula III defined in claim 31, to obtain a compound of formula VIII:

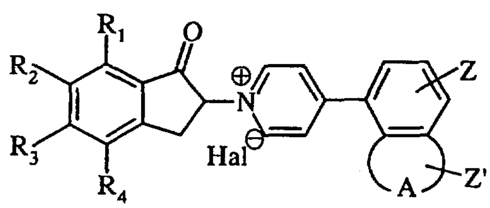

(VIII)

wherein $R_1$, $R_2$, $R_3$, $R_4$, A, Z, Z' and Hal are as defined in claim 31,
- which is reduced with a metal borohydride in an alcoholic solvent to yield a mixture of amino alcohols of formulae I'a *trans* and I'a *cis* :

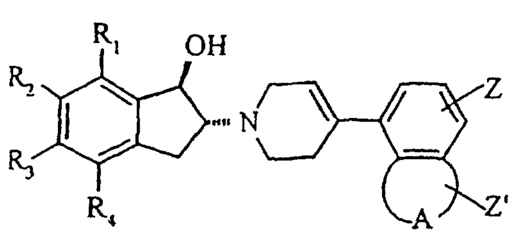

(I'a trans)

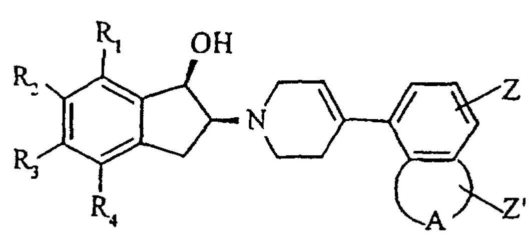

(I'a cis)

wherein $R_1$, $R_2$, $R_3$, $R_4$, A, Z and Z' are as defined hereinbefore,
- which amino alcohols are, if desired, reduced again, with hydrogen in the presence of a suitable catalyst, to compounds of formulae I"a *trans* and I"a *cis* :

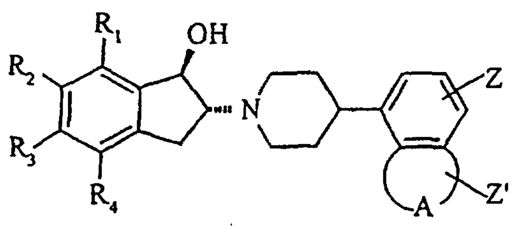

(I"a trans)

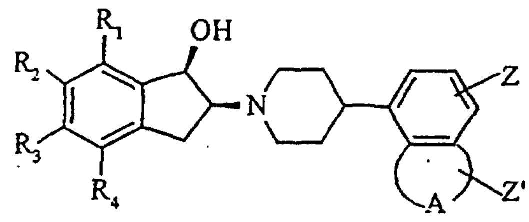

(I"a cis)

wherein $R_1$, $R_2$, $R_3$, $R_4$, A, Z and Z' are as defined hereinbefore,

- and, when Z' represents oxo, it is advantageous to start from a compound VII in which the oxo function is first protected and then, at the stage of the compounds I'a *trans* and I'a *cis* or at the stage of the compounds I"a *trans* and I"a *cis*, deprotected,
- the compounds I'a *trans* and I'a *cis* on the one hand and the compounds I"a *trans* and I"a *cis* on the other hand are then separated by conventional methods for separating organic compounds,

and, if desired:

- the racemic forms are separated to obtain the corresponding optically active forms, and/or
- the resulting compounds Ia are converted into salts with pharmaceutically acceptable acids to obtain the corresponding salts.

**33.** Pharmaceutical compositions comprising as active ingredient at least one compound according to any one of claims 1 to 30, mixed or in association with one or more inert, non-toxic, pharmaceutically acceptable excipients or carriers.

**34.** Pharmaceutical compositions according to claim 33 comprising at least one active ingredient according to any one of claims 1 to 30, for use in the treatment of diseases of the central nervous system or of manifestations of pain.